# EUROPEAN PATENT SPECIFICATION

(11) **EP 1 703 923 B1**
(45) Date of publication and mention of the grant of the patent: **07.05.2008**
(21) Application number: 04815131.0
(22) Date of filing: 17.12.2004
(51) Int. Cl.: A61M 3/02, A61B 10/00

(54) **MEDICAL INSTRUMENT FOR ACCESSING A BREAST DUCT FOR PERFORMING A MEDICAL PROCEDURE**
MEDIZINISCHES INSTRUMENT ZUM ZUGRIFF AUF EINEN MILCHGANG ZUR DURCHFÜHRUNG EINES MEDIZINISCHEN VORGANGS
INSTRUMENT MEDICAL D'ACCES A UN CANAL GALACTOPHORE POUR LA REALISATION D'UNE INTERVENTION MEDICALE

(30) Priority: 23.12.2003 US 746128; 23.12.2003 US 746950; 23.12.2003 US 746940; 23.12.2003 US 746121; 23.12.2003 US 746117
(43) Date of publication of application: 27.09.2006
(73) Proprietor: CYTYC CORPORATION, Marlborough, MA 01752 (US)
(72) Inventor: SAKAL, Robert, Bolton, MA 01740 (US); BRENNAN, Meghan, Newburyport, MA 01950 (US); FURNISH, Greg, R., Louisville, KY 40206 (US); WHATLEY, Charles, H., Louisville, KY 40207 (US); MORRIS, Ben, E., Louisville, KY 40205 (US); WOOD, Nathan, Winchendon, MA 01475 (US); MACARTHUR, Douglas, Acton, MA 01720 (US); HALL, Todd, A., Goshen, KY 40026 (US); FURNISH, Simon, New York, NY 10009 (US); SHEETS, Ellen, Concord, MA 01742 (US); GUNASEKARA, Indi, Louisville, KY 40220 (US); SMTH, Arthur, L., Tyngsborough, MA 01879 (US)
(74) Representative: Tombling, Adrian George
(86) International application number: PCT/US2004/043015
(87) International publication number: WO 2005/063321

(56) References cited:
- WO-A-95/01198
- WO-A-02/083005
- WO-A-03/080167
- US-A- 2 874 694
- US-A- 5 683 420
- US-A1- 2001 001 117
- US-A1- 2002 029 031
- US-A1- 2003 187 411
- US-B1- 6 592 544

## Description

### FIELD OF THE INVENTION

The present invention relates to a medical instrument having at least a portion that is introduced into the body of a mammal in order to perform diagnostic or therapeutic medical procedures, more specifically, the present invention relates to a medical instrument useable during a diagnostic or therapeutic medical procedure that has a low profile and at least a portion sized for introducing into a breast duct through a ductal orifice.

### BACKGROUND OF THE INVENTION

The breast is a specialized, glandular structure including a system of complicated breast ducts that radiate from the nipple and that are bound together by fairly dense connective tissue. Each of these breast ducts includes an associated ductal orifice on the surface of a nipple through which ductal fluid may be expressed. Each duct includes a series of successive interlobular branches that drain through the main, lactiferous branch, which terminates and exits the breast at the nipple via the associated ductal orifice. Immediately proximate the ductal orifice, each lactiferous duct includes a lactiferous sinus in which ductal fluid may accumulate. A ductal sphincter resides within the lactiferous sinus and prevents ductal fluid from unintentionally exiting the breast duct through its associated ductal orifice.

Breast cancer is believed to begin in the lining of these breast ducts. For several decades significant members of the medical community dedicated to studying breast cancer have believed and shown that the cytological analysis of cells retrieved from nipple discharge fluid from within breast ducts may provide valuable information leading to identifying patients at risk for breast cancer. Indeed, Papanicolaou contributed to the genesis of such a possibility of a "Pap" smear for breast cancer by analyzing the cells contained in nipple discharge. More recently, cancer specific markers have been detected in ductal fluid obtained by nipple aspiration. However, the retrieval techniques and instruments used by these members of the medical community did not routinely obtain meaningful ductal fluid samples.

In their attempts to retrieve the breast duct fluid sample including ductal epithelial cells, practitioners introduced wash fluids into a breast duct using indwelling hair-like single lumen catheters. After the fluid was introduced into the duct, the fluid introduction catheters were removed. Then, externally applied nipple aspiration techniques or external pressure applied to the breast were used to collect samples of the ductal fluid. However, these techniques required that significant, sometimes painful, pressure be created on the nipple surface or along the sides of the breast to overcome the fluid retaining properties of the ductal sphincter. Also, these techniques did not routinely provide meaningful ductal fluid samples with a sufficient number of ductal epithelial cells for a meaningful cellular analysis. These techniques typically caused the recovery of samples with twenty or fewer ductal epithelial cells. Additionally, these techniques did not provide samples with cell clusters of 10 or more cells. As a result, the obtained fluid samples could not consistently provide an accurate indication of whether or not the duct from which they were retrieved included precancerous or cancerous cells. Consistent, meaningful ductal epithelial cell samples have been provided by the medical instrument disclosed in U.S. Patent No. 6,413,228 to Hung et al..

Other medical instruments, such as those used during galactography, are introduced into the breast duct in order to visually determine the presence of cancerous cells within a breast duct. However, these devices typically extend a significant distance out of the breast duct during the performed procedure. These distances may be twelve inches or greater. As a result, when an operator is not holding the tool, the moment created by the weight and length of the section of the instrument extending out of the duct may cause the indwelling portion of the instrument to engage the sidewalls of the duct, torque and/or kink the duct and distort the nipple. These effects on the duct and nipple may impede the procedure by twisting or crimping the indwelling portion of the instrument, possibly injuring the patient's duct and causing significant discomfort to the patient. As a result, a patient must either endure the pain and discomfort caused by these long instruments or an attendant must constantly support the instrument above the patient during the medical procedure. However, in the confined space around an operating table and in the area surrounding a nipple surface, it is not practical to have an attendant constantly holding the end of the instrument that is extending into the breast duct. Therefore, prior to receiving the procedure, a patient must decide to either experience discomfort during the procedure or choose not to have the procedure performed. Prior art instruments are also not ergonomically designed for easy grasping and adjusting by a practitioner or attendant while acting in the area surrounding a nipple.

Patients with tight ductal sphincters or tortuous ductal orifices may experience difficulties with the lavage procedure due to twisting or crimping the indwelling portion of the catheter, possibly injuring the patient's duct and causing significant discomfort to the patient Thus, improved methods for accessing breast ducts with minimal discomfort to the patient are needed.

The human breasts are composed of fatty tissue, fibrous tissue, breast ducts and milk glands. Human breasts are believed to contain from 6 to 8, or more breast ducts. The ductal lavage procedure discussed above, and sampling results may be greatly effective in screening patients for an early warning of breast cancer risk. However, in performing the ductal lavage procedure, a physician may have difficulty inserting the catheter into a breast duct The breast duct is a complex anatomical pathway to the breast milk glands. The physician must access the breast duct so as not to cause damage to the inner walls of the duct and/or avoid puncturing the duct However, it is believed the deeper a catheter is inserted into breast duct, the greater the risk of puncturing the breast duct walls. Therefore, a need exists for a ductal access catheter that allows the physician to adjust its flexibility and rigidity so as to adapt the catheter to the ductal geometry and direct the catheter deep into branches of the ductal network.

During the ductal access procedure, a catheter is inserted into a duct opening in the nipple that may cause some discomfort to the patient Thus, improved insertions systems and methods for ductal access are needed.

International Patent Application WO 02/083005 discloses a device for accessing a mammalian duct comprising a manifold hub and a catheter extending from said manifold hub, wherein the length (direction perpendicular to the longitudinal axis of the catheter) of said manifold hub is smaller than the height (direction parallel to the longitudinal axis of the catheter) of said manifold hub.

### SUMMARY OF THE INVENTION

In accordance with one aspect of the present invention, a medical device for introducing a fluid into a breast duct is provided. The medical device comprises a catheter that is sized and configured to extend within a breast duct. The catheter includes an internal lumen that extends substantially parallel to a longitudinal axis of the catheter. The medical device also comprises a manifold hub connected to and in fluid communication with the catheter. The manifold hub includes at least four ports in fluid communication within an interior of the manifold hub. At least two of the at least four ports are in fluid communication with channels formed within the manifold hub for receiving a fluid introduction line and a ductal fluid collection line. The manifold hub has a height that extends parallel the longitudinal axis of the catheter and a length that extends perpendicular to the longitudinal axis of the catheter. The length of the manifold hub is greater than the height of the manifold hub.

A catheter in accordance with the present invention may have an outer diameter of about 0.01 inch (0.254 mm) to about 0.05 inch (or 1.27 mm). The catheter may have an inner lumen diameter in the range from about 0.007 inch (or 0.178 mm) to about 0.047 inch (or 1.19 mm). The associated manifold hub may further comprise an infusion connector providing a fluid flow path into the lumen of the catheter from an infusion device; and a collection connector providing a fluid outlet path from the lumen of the catheter to a fluid collection device.

A watertight sealing system may be located at a proximal end of the manifold hub. This sealing system may seal around a dilator or other introducer positioned within and extending through the medical instrument. Such a sealing system may include a Touhy-Borst fitting. A dilator or other introducer member(s) for use with the catheter may have an outer diameter of 0.024 inch (or 0.61 mm) to about 0.001 inch. The introducer may also be tapered.

### BRIEF DESCRIPTION OF THE FIGURES

Figure 1 is a perspective view of a medical instrument according to aspects of the present invention;

Figures 2A and 2B are cross sections of alternative embodiments of the medical instrument illustrated in Figure 1 that do not form part of the invention;

Figure 3 is an exploded perspective view of the medical instrument of Figure 1;

Figure 4 is a top view of the medical instrument of Figure 1 with infusion and collection lines extending between a manifold hub and respective infusion and collection devices;

Figure 5 is a side view of the medical instrument illustrated in Figure 1 carrying infusion and collection lines;

Figure 6 is another perspective view of the medical instrument illustrated in Figure 1;

Figures 7 and 8 illustrate an alternative embodiment of the medical instrument according to aspects of the present invention;

Figures 9-10 illustrate a method for introducing the medical instrument of Figure 1 into a breast duct using a stiff introducer; and

Figures 12-14 illustrate an alternative method for introducing the medical instrument of Figure 1 into a breast duct using a flexible introducer.

### DETAILED DESCRIPTION OF THE INVENTION

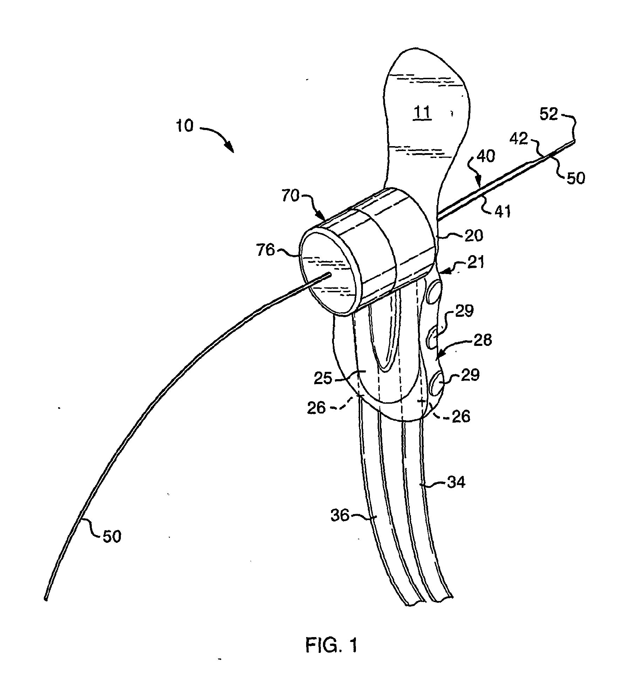

Figure 1 illustrates an embodiment of a low profile, single lumen medical instrument 10 for performing a medical procedure within a breast duct. As used herein, the phrase "medical procedure" may include preparatory procedures, diagnostic procedures or therapeutic procedures. These procedures could include the steps of delivering material(s) into the breast duct and/or retrieving material(s) from within the breast duct.

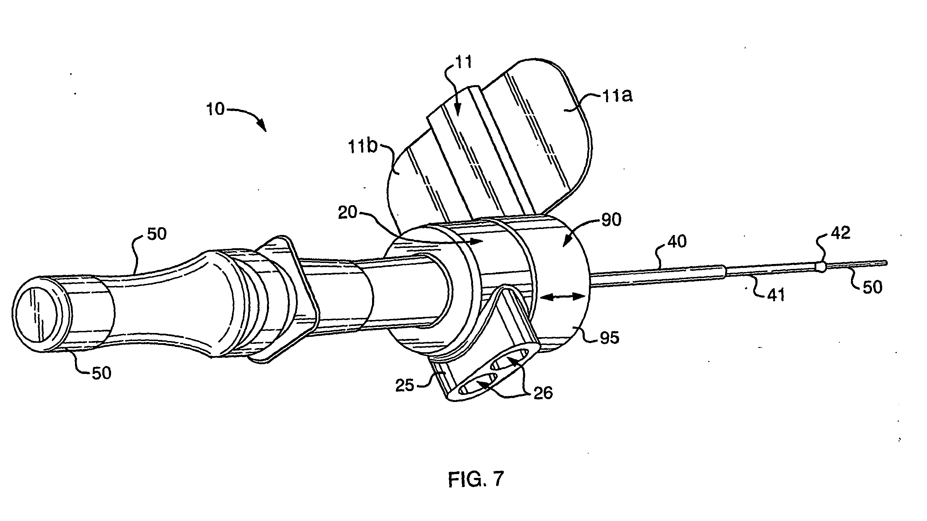

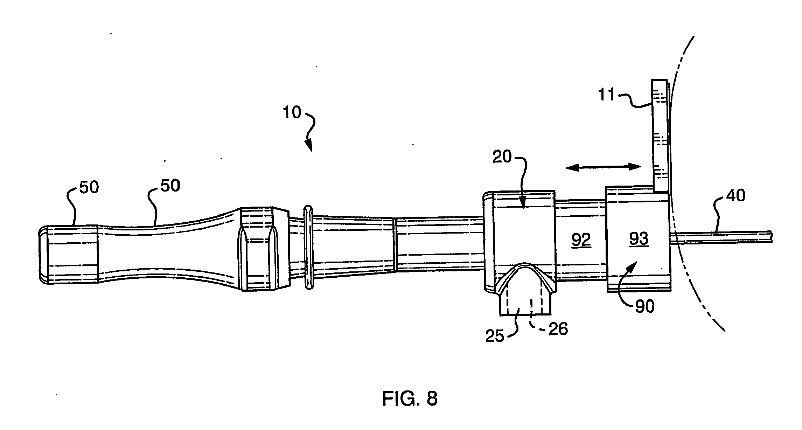

The medical instrument 10 may be used to infuse ductal wash fluid delivered to the manifold hub 20 into the breast duct, and collect or draw up ductal fluid samples, including hundreds of ductal epithelial cells and/or cell clusters of greater than ten cells, from within the breast duct for analysis. Furthermore, the medical instrument 10 may be used to infuse a diagnostic agent or therapeutic agent into a breast duct. As shown in Figures 1-8, the instrument 10 may also include a member 11 for securing the instrument 10 to a patient. The securing member 11 may have a biocompatible adhesive on one side for contacting and attaching the instrument 10 to the patient. The member 11 is sized to prevent movement of the manifold hub 20 relative to the body of the patient. As illustrated in Figures 7 and 8, sections 11A and 11B of the member 11 may be folded onto each other so that the size of the securing member 11 may be adjusted to the patient and the forces created during the procedure. Additionally, the member 11 may be positioned distal a spacer 90 (discussed below).

As illustrated in Figures 1-6, the medical instrument 10 includes a manifold hub 20 and a ductal access catheter 40 that extends from a distal end 21 of the manifold hub 20. The access catheter 40 is sized to accesses the breast duct. As illustrated, the manifold hub 20 has a low profile (height) in a direction that extends parallel to the length of the catheter 40. As illustrated in Figures 1 and 3 to 6 the height of the manifold hub 20 is less than its length (the direction it extends perpendicular to the length of the catheter 40). In a first embodiment, the manifold hub 20 may have a width in a range from about 6.35 mm (0.25 inch) to about 9.525 mm (0.375 inch) and a height in a range from about 19.05 mm (0.75 inch) to about 25.4 mm (1.0 inch). In another construction, the manifold hub 20 has an internal fluid capacity of 1 ml or less. The low profile of the manifold hub 20 will help to prevent a pivot point from being formed at a location along the length of the catheter 40 at which a large torque may be applied to the duct of a patient during a medical procedure. By eliminating or, at least, significantly reducing any torquing of the duct, the duct will not be kinked, closed due to a change in the position of ductal tissue or injured due to the catheter 40 pushing against the epithelial lining of the duct. The instrument 10 also has an ergonomic design that allows easily handling and grasping by an attendant or practitioner so that the manifold hub 20 and catheter 40 may be easily manipulated.

In the event that the manifold hub 20 needs to be spaced from the nipple surface, a retractable spacer 90 may be positioned on the distal end of the manifold hub and at the proximal end of the catheter 40 as shown in Figures 7 and 8. The retractable spacer 90 may have a spacing distance in the range from about 1mm to about 10mm, most typically in the range of about 5mm. In a first embodiment, the retractable spacer 90 may include a first spacing member 92 received within a second spacing member 93. Alternatively, the first spacing member 92 may telescopically receive the second spacing member 93. In this construction, the first member 92 is secured against movement relative to the manifold hub 20, while the second member 93 is moveable relative to both the first member 92 and the manifold hub 20. Alternatively, both of the spacing members 92, 93 are moveable relative to the manifold hub 20. The distances required for spacing the manifold hub 20 from the surface of the nipple, for example distances of between about 15 mm and 20 mm, may be controlled by locking the spacing members 92, 93 in an extended position (Figure 8). Alternatively, the retractable spacer 90 may be locked in a retracted position (Figure 7). Alternatively, the retractable spacer 90 includes a single moveable spacing member 95 that slidably receives a portion of the manifold hub 20 to achieve the retracted position and that may be locked at the end of the manifold hub 20 to achieve the extended position. In any of the above-discussed embodiments, the spacing members 92, 93, 95 may be rotated relative to each other and the manifold hub 20 in order to lock each spacing member 92, 93 and 95 against translational movement relative to each other and the manifold hub 20. Any known rotational locking system for telescoping members may be used. Alternatively, the spacing members 92, 93, 95 may be snapped into a locked position using well known snap locks. When additional spacing is needed, members 92, 93, 95 of different sizes or more than two telescoping members may be provided.

The manifold hub 20 may be formed of a transparent material so that an attendant or practitioner may easily view fluid(s) and material(s) within the manifold hub 20. The transparent material may be a plastic, such as ABS plastics or other known plastic materials. As illustrated in Figures 1-8, an embodiment of the manifold hub 20 may have a substantially "F" shape.

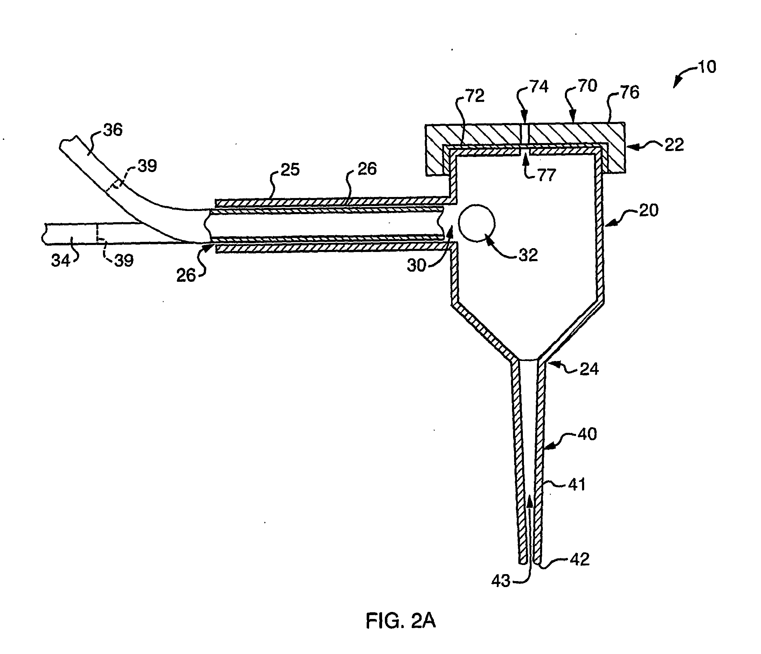

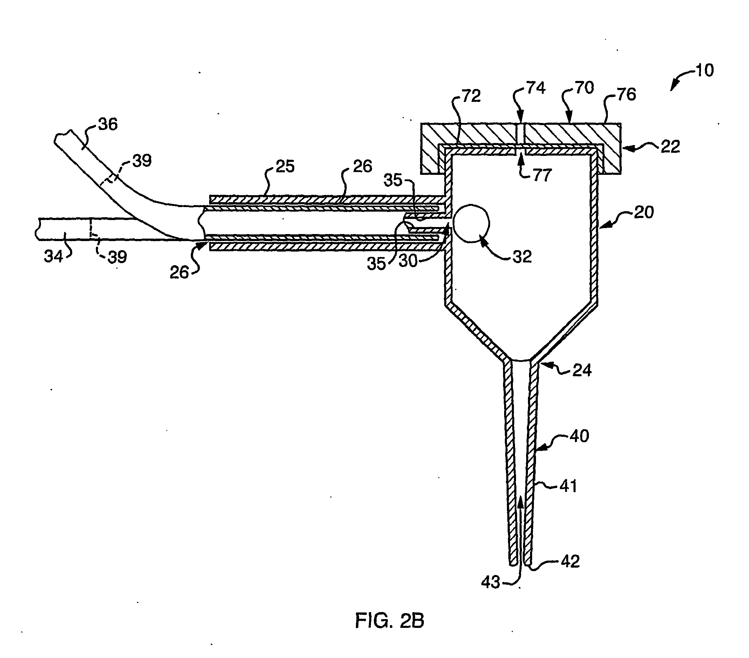

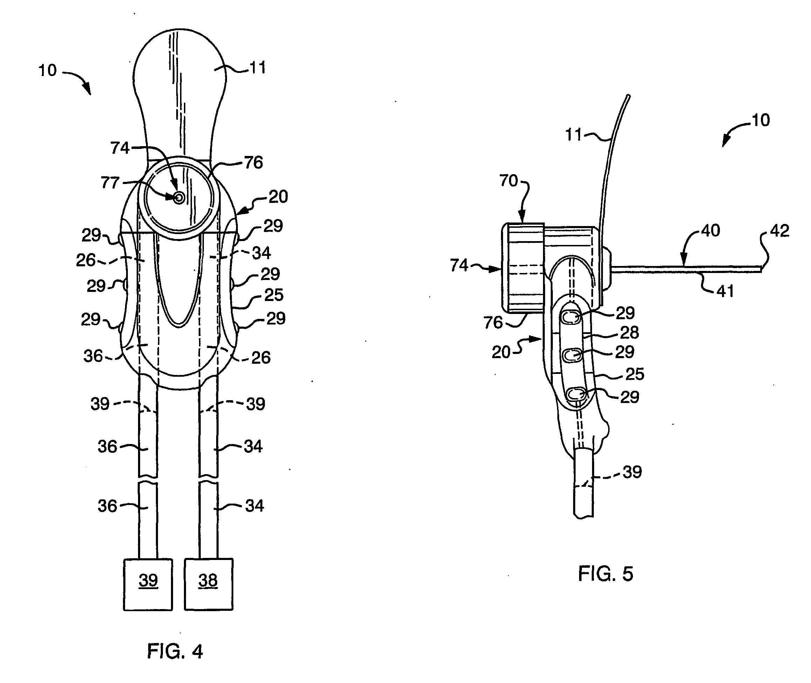

As shown in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. The connected infusion device 38 may include a syringe or other known fluid containers. The infusion device 38 may include a fluid receptacle, such as a bag or a container, positioned at a location above the breast of the patient. The height of the container above the breast of the patient and gravity are used to deliver the fluid from the infusion device 38 to the infusion tube 34. This embodiment does not form part of the present invention.

As shown in Figures 2A, 2B and 4, the manifold hub 20 also includes a second port 32 for connection to a collection tube 36. Ductal fluid samples collected from within the breast duct may be delivered from the manifold hub 20 to a collection receptacle via the collection tube 36. The collection receptacle may include a syringe or other known fluid collection device including a medical fluid bag or container. The collection receptacle may include or be connected to a source of negative pressure so that an area of low pressure may be created within the collection tube 36 and, if needed, the manifold hub 20 for assisting in the delivery of the retrieved ductal fluid sample to the collection receptacle. The area of low pressure, for example a vacuum, may be created using a bulb syringe, a hand-operated vacuum source, a foot operated vacuum source or motor controlled vacuum source. These vacuum sources may include a pump that creates negative pressure within the collection tube 36. In addition to a source of lower pressure, or in place of the source of lower pressure, low pressure within collection tube 36 may be created by infusing fluid into the manifold hub 20, thereby increasing the pressure within the manifold hub 20 relative to the collection tube 36. In any of these construction, the collection receptacle may be positioned at a location below the patient during the procedure so that the collected ductal fluid sample may be delivered to the receptacle by gravity.

The first and second ports 30, 32 may include an opening along the sidewall of the manifold hub 20 that is round, oval or any other geometric shape conductive to fluid flow either into the duct or out from the duct as shown in Figures 2A and 2B. The diameter of the ports 30, 32 may be that diameter which is suitable to achieve a desired flow rate into the duct or aspiration or collection rate out from the duct. Thus, the diameters of the ports 30, 32 may be in a range from about 1.52 mm (0.060 inches) to about 2.29 mm (0.090 inches). One side port 30, 32 may be larger or smaller than the other, especially where such differential port size provides a desired flow rate into or out from one of the lumens, or an overall lavage efficiency of infusion and aspiration or collection of lavage and ductal fluid. The embodiment shown in Figures 2A & 2B do not form part of the present invention.

Figures 1-6 illustrate that the first and second ports 30, 32 and their associated tubes 34, 36, respectively, are positioned within a connector housing 25 that extends transverse to the longitudinal axis of the manifold hub 20 and the catheter 40. The connector housing 25 may be integrally formed with the manifold hub 20 as a unitary element. Alternatively, the connector housing 25 may be formed separate of the manifold hub 20 and secured to the manifold hub 20 as discussed below with respect to the catheter 40. The connector housing 25 includes two channels 26 that each receives one of the tubes 34, 36. The channels 26 extend from the ports 30, 32 on the manifold hub 20 to the outer, end surface of the connector housing 25. Each channel 26 aligns the received tube 34, 36 with its respective manifold hub port 30, 32 for easy, reliable and quick connection of the tubes 34, 36 to their respective ports 30, 32. The channels 26 also support the tubes 34, 36 at their point of connection to their ports 30, 32 so that the tubes 34, 36 do not create a moment (that may torque the duct) about the point where they connect to their respective ports 30, 32. The connector housing 25 may also include contoured sidewalls 28 with integrally formed, or otherwise secured, ridges 29 that may be gripped by an attendant or a practitioner. The contoured sidewalls 28 permit easy grasping by the attendant or practitioner and allow the attendant or practitioner to orient the instrument 10 during a procedure without looking at the instrument 10.

As shown in Figure 2B, the first and second ports 30, 32 may include posts 35 that extend within connector housing 25 and receive the flexible infusion and collection tubes 34, 36. The infusion and collection tubes 34, 36 may be formed of flexible tubing such as surgical tubing. However, the tubes 34, 36 may be formed of any flexible material including a flexible plastic. The tubes 34, 36 are formed of flexible PVC. The embodiment shown in Figure 2B does not form part of the present invention.

The posts 35 may securely receive the tubes 34, 36 when the tubes 34, 36 are positioned over, or within, the posts 35. Alternatively, the ports 30, 32 and their associated posts 35 may include luer lock fittings (not shown) that cooperate with corresponding luer lock fittings on a first end of the tubes 34, 36. The second end of the tubes 34, 36 may also include luer lock fittings that mate with standard luer lock fittings on the syringes or other fluid containers. The luer lock fittings may be either male or female fittings. As discussed herein, the syringes and other fluid containers may carry and infuse saline, diagnostic materials, such as contrast materials, and therapeutic treatment materials into the infusion tube 34. The tubes 34, 36 may be secured to the posts 35 of the manifold hub 20 and the luer locks using a UV curable adhesive or other known bonding agents. Alternatively, the tubes 34, 36 may be secured to the manifold hub 20 and the luer locks by overmolding.

As shown in Figures 1-6, the first port 30 may be positioned adjacent the second port 32 along the perimeter of the manifold hub 20. In the illustrated embodiment, the circumferentially adjacent ports 30, 32 are spaced the same longitudinal distance from the first and second ends 22, 24 of the manifold hub 20. This spacing of the ports 30, 32 provides for a compact and low profile manifold hub 20 that, as discussed above, will not create a moment and associated forces that toque the duct when the manifold hub 20 is positioned on the patient and free of support from a practitioner or other attendant.

The second port 32 may be circumferentially spaced any distance from the first port 30 around the wall of the manifold hub 20. The first port 30 may be between forty-five and ninety degrees offset from the second port 32 around the circumference of the manifold hub 20. Alternatively, the first port 30 may be circumferentially offset along the manifold wall from the second port 32 by between ninety and one hundred-eighty degrees. The first port 30 is circumferentially offset from the second port 32 by about one hundred-eighty degrees so that the first and second ports 30, 32 oppose each other within the manifold hub 20.

Alternatively, it is possible for the second port 32 to be located along the hub 20 at a position that is spaced a greater longitudinal distance away from the catheter 40 than the first port 30. The second port 32 may be used to collect the fluid that enters the manifold hub 20 from the collection catheter 40. For example one port may be located about 2.0 cm from the distal tip of the catheter 40 and one port may be located about 2.5 cm from the distal tip of the catheter 40.

The tubes 34 and 36 may each include a one-way check valve 39 (Figure 2A) to control the fluid flow into and out of the manifold hub 20. The check valve 39 in the tube 34 may prevent, for example, wash fluid from flowing back into a syringe connected to tube 34 after being infused into tube 34. Similarly, check valve 39 in tube 36 may be used to prevent retrieved ductal fluid samples in tube 36 from flowing back into the manifold hub 20. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used. The embodiment shown in Figure 2A does not form part of the present invention.

As shown in Figures 1-6, the catheter 40 includes a thin walled microcatheter 41 that is secured to the manifold hub 20. In a first construction, the microcatheter 41 is integrally formed as part of the manifold hub 20. In another construction, the microcatheter 41 is formed as a separate piece and then secured to the manifold hub 20 by microwelding or a UV curable adhesive. Other known techniques for securing the microcatheter 41 to the manifold hub 20 could be used. Moreover, the catheter 40 may be coated with a known agent to provide a lubricious coating that allows it to be easily introduced into the breast duct openings. The coating may include a lubricant, a cleaning agent, anesthetic and/or a dilating agent. The microcatheter 41 may be formed of any known biocompatible material such as FEP. The catheter 40 may have an outer diameter in a range from about 0.01inch (about 0.25 mm) to about 0.05 inch (about 1.25 mm) with an inner lumen 43 having a diameter in the range from about 0.008 inch (about 0.2 mm) to about 0.047 inch (about 1.2 mm). Furthermore, the microcatheter 41 may have an inner lumen 43 having an outer diameter of about 0.030 inch (about 0.762 mm) and an inner diameter of about 0.025 inch (about 0.63 mm).

The catheter 40 may include length indicia (not shown) on an outer surface of the catheter 40 that permits a user to determine the depth to which the distal end of the catheter has been introduced into the breast duct. Alternatively, the catheter 40 could include an integrally formed or attached stop element (not shown) that prevents insertion of the catheter into the duct beyond a predetermined distance. The stop element may comprise a knob on the catheter 40 having an increased diameter for preventing the distal portion of the catheter 40 from entering a duct a greater distance than the knob is spaced from the distal end of the catheter40.

As illustrated in Figures 1-7, the catheter 40 may be tapered along its length to make a smooth transition with a received introducer 50 so that a perceptible transition between the catheter 40 and the introducer 50 that would cause any pain to the patient is not formed and felt by the patient. The catheter 40 may also include an atraumatic distal tip portion 42 at its distal end. The distal tip portion 42 may be tapered, contoured and/or rounded so as to produce an traumatic tip that will reduce or eliminate trauma to the duct upon entry through the ductal orifice and introduction into the ductal lumen past the ductal sphincter. The distal tip portion 42 may also reduce or eliminate trauma upon withdrawal of the catheter 40 from the duct after the medical procedure, such as ductal lavage or the infusion of a diagnostic and/or treatment agent, has been completed. The tip portion 42 may be composed of a soft polymeric material, e.g. including polyvinyl chloride, polyethers, polyamides, polyethylenes, polyurethanes, copolymers thereof and the like. The tip portion 42 may have a diameter in the range from about 0.012 inches (about 0.31 mm) to about 0.020 inches (about 0.51 mm). In a construction, the tip portion 42 has a diameter in the range from about 0.014 inches (about 0.36 mm) to about 0.018 inches (about 0.46 mm). The length of the tip portion 42 (extending from the distal end of the distal portion of the catheter 40 toward the proximal end of the catheter 40) may be in a range from about 0.10 inch (about 0.25 cm) to about 1.0 inch (about 2.5 cm), more typically in the range from about 0.20 inch (about 0.50 cm) to about 0.70 inch (about 1.8 cm).

The stiffened distal portion of the catheter 40, including the distal tip 42, may have an average bending stiffness in the range from about 0.001 cm-kg (0.010 inch-lbs) to about 0.576 cm-kg (0.5 inch-lbs). The catheter 40 may also have a stiffness that is similar to that of a heavy suture (approximately 0.025 OD). The proximal portion of the catheter 40 may have a cross-sectional geometry and/or size that inhibits insertion through the ductal orifice and into the ductal lumen.

A Touhy-Borst fitting 70 may be positioned at a proximal end 22 of the manifold hub 20 to allow a user to easily receive and move the catheter 40 over an introducer 50 as shown in Figures 1-6. The Touhy-Borst fitting 70 is positioned at the end of the manifold hub 20 to cover and seal the opening 77 through which an introducer 50 (discussed-below) including a guidewire, stylet, dilator or the like may extend. The Touhy-Borst fitting 70 comprises a silicone plug 72 including a small aperture 74 for receiving the introducer 50, and a threaded cap 76. When the cap 76 is rotated in a first direction, the silicone plug 72 is altered and the size of the aperture 74 is reduced. This results in the silicone plug 72 forming a seal around the inserted introducer 50. When the cap 76 is turned in a second, opposite direction, the aperture 74 and created seal open, thereby allowing the introducer 50 to be removed. The silicone plug 72 may also be closed to seal the proximal end of the manifold hub 20 so when the introducer 50 is not present so that the distal end of the manifold hub 20 is sealed against fluid flow when the proximal end 22 is free of an introducer 50.

The introducer 50 may be located within the manifold hub 20 to assist in placing the catheter 40 into the breast duct and ductal lumen via the ductal opening as shown in Figure 1. The introducer 50 may include a tapered dilator, a series of progressively larger diameter dilators, a guidewire, including tapered guidewires, a stylet or other known introducers. As illustrated, the introducer 50 will pass through the Touhy-Borst fitting 70 at the proximal end 22 of the manifold hub 20 so that the introducer 50 may be removed after positioning of the catheter 40 and prior to the infusion/collection of the wash fluid. As discussed above, prior to being inserted into the breast duct, the Touhy-Borst fitting 70 may be turned down over the introducer 50 during introduction and then backed off when the catheter 40 has been positioned within the breast duct to the desired depth. The introducer 50 may be formed of a stiff material such as a metal wire or a flexible plastic cord. Furthermore, the introducer 50 may be formed of stainless steel or a flexible material such as polypropylene monofilament. Alternatively, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. The introducer 50 may be coated with a liquid or dry lubricant material that reduces the friction between the introducer 50 and the breast duct during the introduction and advancement of the introducer 50 in the duct

The introducer 50 may be made of metal or plastics, including shape memory metals and plastics, and may have a tapered and/or an atraumatic tip for gently probing and accessing a breast duct. Preferably, a tapered tip 52 will extend distally of the catheter 40 during the introduction of the catheter 40 into the breast duct. After access of the duct is complete, the introducer 50 may be withdrawn, the Touhy-Borst fitting 70 may be closed and the indwelling catheter 40 may be positioned at a location distal to the ductal sphincter. The introducer 50 may have an outer diameter of from about 0.13 mm (0.005 inch) to about 0.76 mm (0.030 inch). In a construction, the introducer 50 has an outer diameter of about 0.25 mm (0.010 inch). The introducer 50 may extend through the manifold hub 20 and the lumen of the catheter 40. The introducer 50 may be tapered over its length.

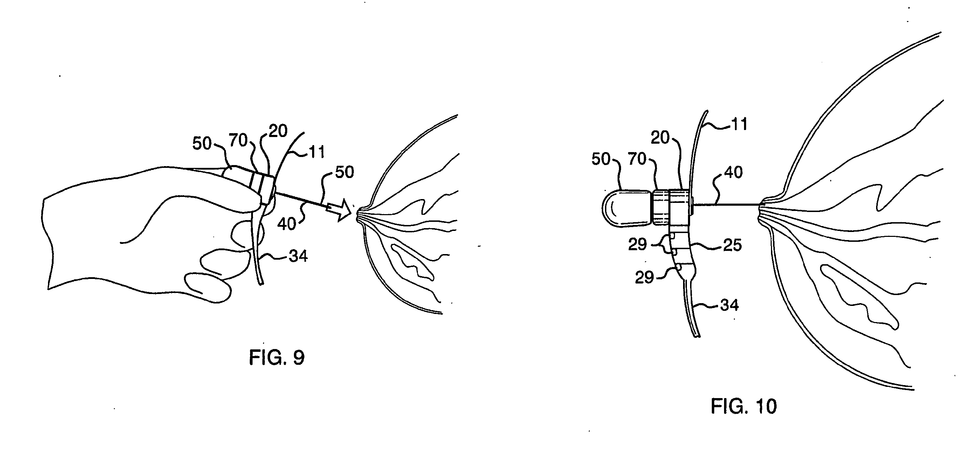

During the process of introducing the catheter 40 into the duct, a ductal opening is located on the surface of a nipple by a practitioner or attendant and a first introducer 50 is advanced through the ductal opening into the duct The introducer 50 may be a long flexible guide wire, a shorter dilator or any of the other above-mentioned introducers. Prior to, or after the introducer 50 is positioned within the duct, the manifold hub 20 and catheter 40 may receive the first or a second introducer 50. As previously discussed, the Touhy-Borst fitting 70 may be locked about the received introducer 50 to form a fluid tight seal at the distal end of the manifold hub 20 so that fluid does not exit the manifold hub 20 around the introducer 50 during the insertion catheter 40 into the duct (See Figures 9, 10, 12 and 13).

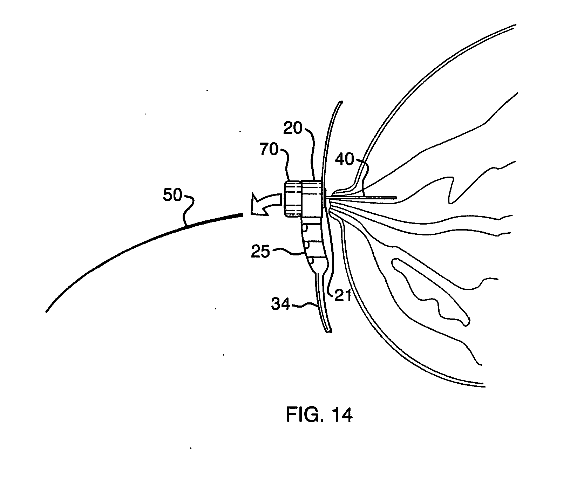

When the catheter 40 is positioned within the duct as intended, the Touhy-Borst fitting 70 is opened and the introducer 50 removed (See Figure 14). The Touhy-Borst 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

A method of lavage may include seating a patient substantially upright in a chair during the lavage procedure, rather than the standard or classic supine (face up) position. Alternatively, the patient may be lavaged in a prone position, face down, with nipples and breast down. The prone face down position takes advantage of gravity and allows the breast ducts to drain into the collection receptacle during the procedure when the outflow port is open. Thus, as discussed above, the lavaging procedure may include infusing the breast duct with a wash fluid through an open inflow lumen while an outflow lumen is closed; closing the inflow lumen when the duct is filled; squeezing or massaging the breast or both; and opening the outflow lumen to collect the wash fluid.

The cells collected may comprise ductal epithelial cells; the ductal fluid collected may comprise molecular and cellular material. Analysis of the ductal epithelial cells and/or the molecular and cellular material in the ductal fluid may proceed as described below discussing the diagnostic methods possible of these collected materials. The collected cells and fluid and fluid components may be analyzed. The lavage fluid including the ductal cells may be analyzed for diagnostic purposes. Conditions in a breast milk duct that are desirable to diagnose include a cancer or precancer condition. The precancer condition may include atypical ductal hyperplasia (ADH) or low-grade ductal carcinoma in situ (LG-DCIS). The diagnostic agent may also have the ability to diagnose other breast related conditions, including, e.g. fibrotic, cystic or conditions relating to lactation. Diagnostic agents may be mixed with the ductal fluid (either in the lavage procedure, or after the fluid is collected).

The fluid infused into the duct to lavage the duct may include known, biocompatible fluids. These lavage fluids may include saline, phosphate buffered saline, a nonabsorbable fluid, an isotonic solution, an osmotic solution, a hypotonic solution, and a hypertonic solution. The wash fluid may comprise for example, saline, phosphate buffered saline, a nonabsorbable fluid, an isotonic solution, an osmotic solution, a hypotonic solution, a hypertonic solution.a protein, a colloid, a sugar, a polymer, mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, a synthetic colloid, an antibody, a binding protein, or albumin.

As mentioned above, a diagnostic or therapeutic agent may be introduced into a breast duct through the manifold hub 20 and catheter 40. The introduced agent for infusing into the duct may comprise a non-absorbable fluid and/or an oncotic agent and/or an osmotic agent. The agent may be soluble. The agent may comprise a molecule that is a protein, a colloid, a sugar, or a polymer. The agent may be mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, or a synthetic colloid. The agent may comprise a protein and the protein may be a binding protein or an antibody. The binding protein may be albumin. Administering may comprise administering locally, and local administration may comprise administering intraductally. A system for increasing or standardizing an amount of fluid collectable from a milk duct of a breast may comprise infusing a nonabsorbable fluid and/or an osmotic agent and/or an oncotic agent into the ductal lumen, a medical tool for delivering the agent to the ductal lumen, and instructions for use.

### DUCTAL LAVAGE CATHETER HAVING AN OFF-AXIS TIP

The device of the present invention may also comprise on off-axis tip.

Other aspects, features and advantages of the present invention will be readily apparent and fully understood from the following detailed description in conjunction with the accompanying drawings, which are included by way of example, and not by way of limitation with regard to the claimed invention.

### BRIEF DESCRIPTION OF THE DRAWINGS

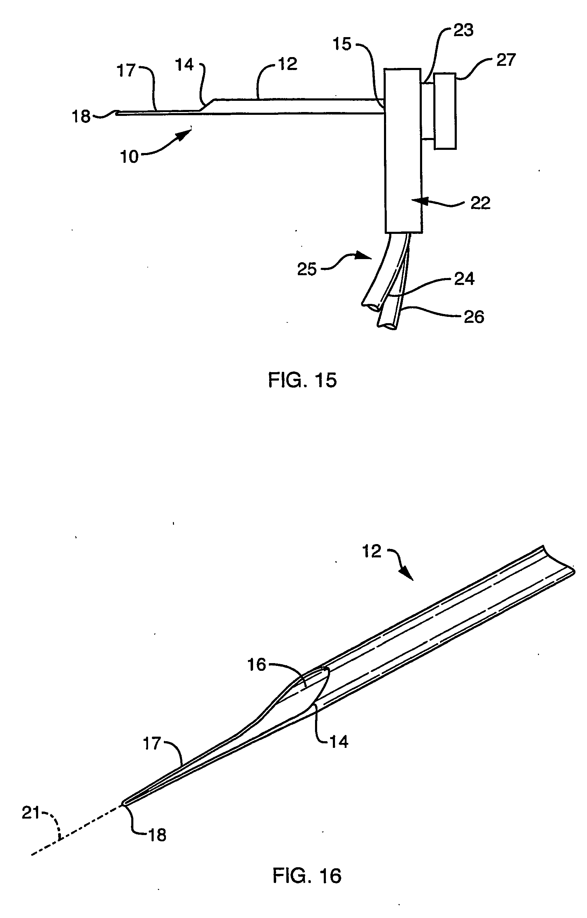

Figure 15 is a schematic representation of a first ductal access device in accordance with the present invention;

Figure 16 is a perspective view of a catheter in accordance with the present invention;

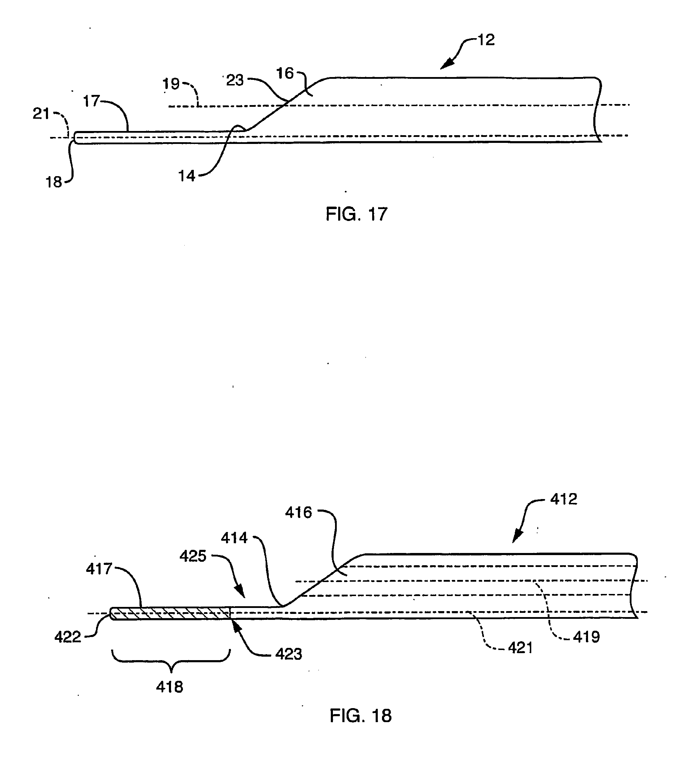

Figure 17 is a partial schematic side view of a catheter in accordance with the present invention;

Figure 18 is a schematic side view of a catheter with a removable tip in accordance with the present invention;

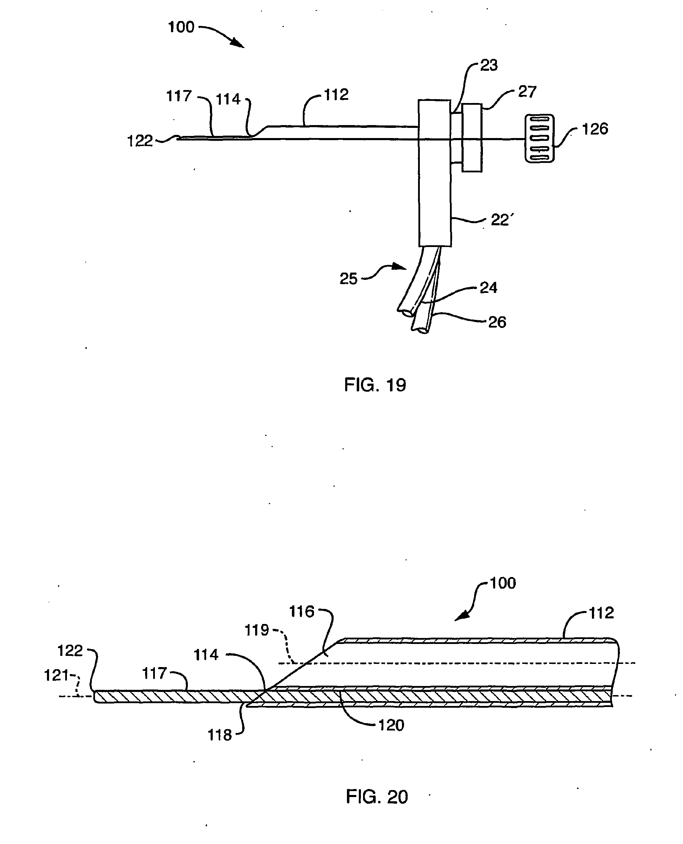

Figure 19 is a schematic representation of a second ductal access device in accordance with the present invention

Figure 20 is a sectional view of a catheter shown in FIG. 19 taken along an central axis to illustrate an interior construction and components;

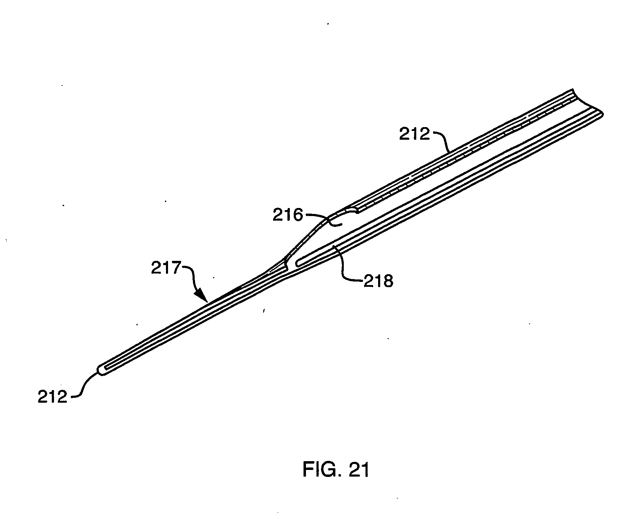

Figure 21 is a perspective view of a catheter;

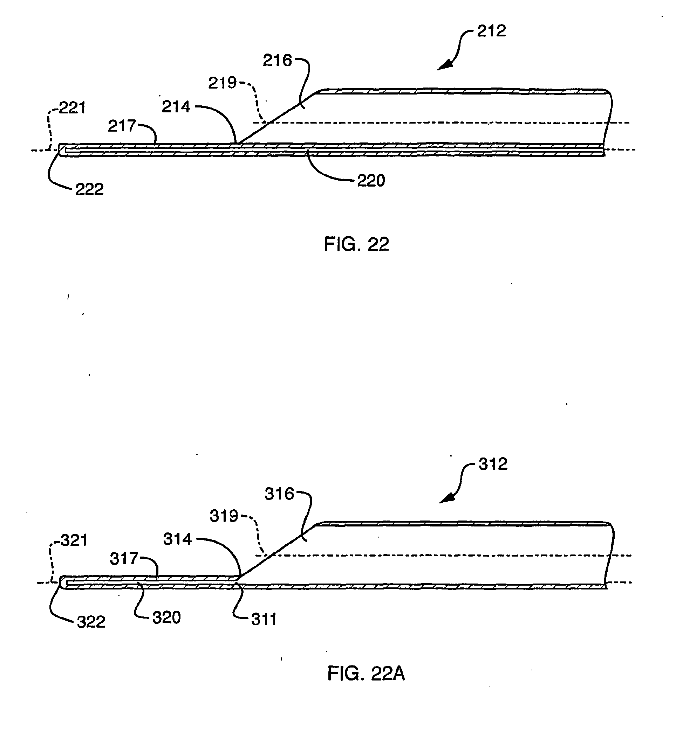

Figure 22 is a sectional view of a catheter shown in FIG. 21 taken along an central axis to illustrate an interior construction and components;

Figure 22A is a sectional view similar to FIG. 22 showing an alterative catheter;

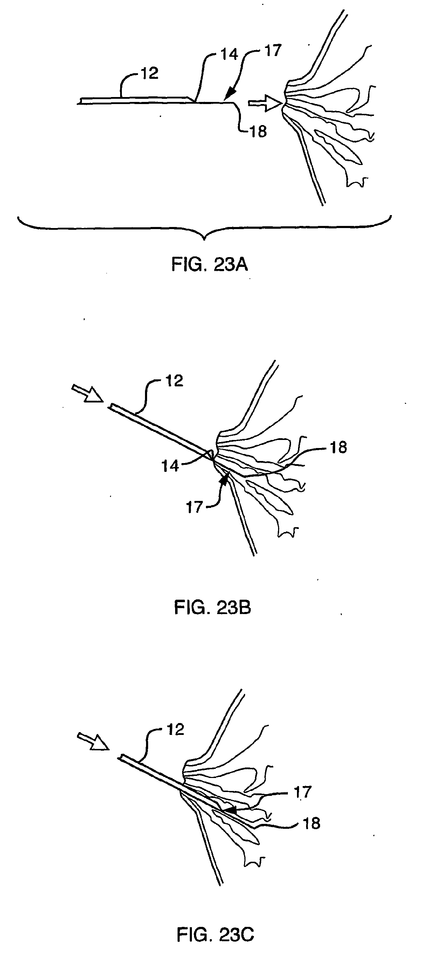

Figures 23A-24C are diagrams illustrating a method of accessing a breast duct which a catheter of FIG. 16;

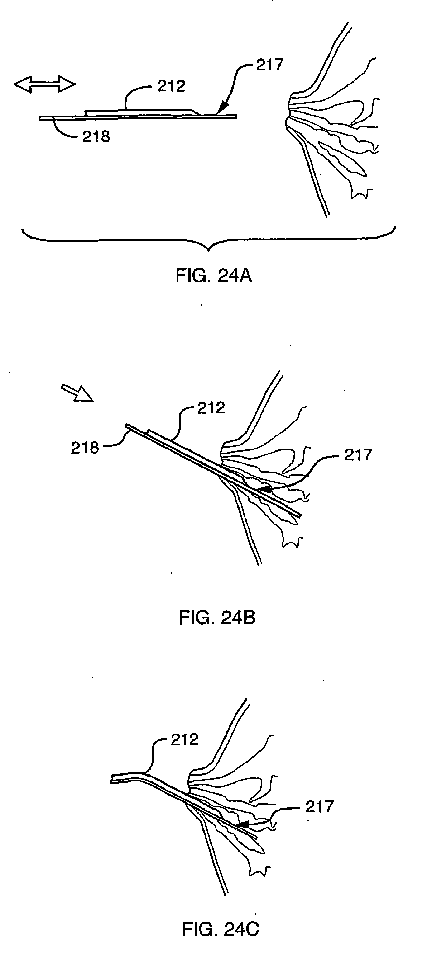

Figures 24A-24C are diagrams illustrating a method of accessing a breast duct which a catheter of FIG. 21;

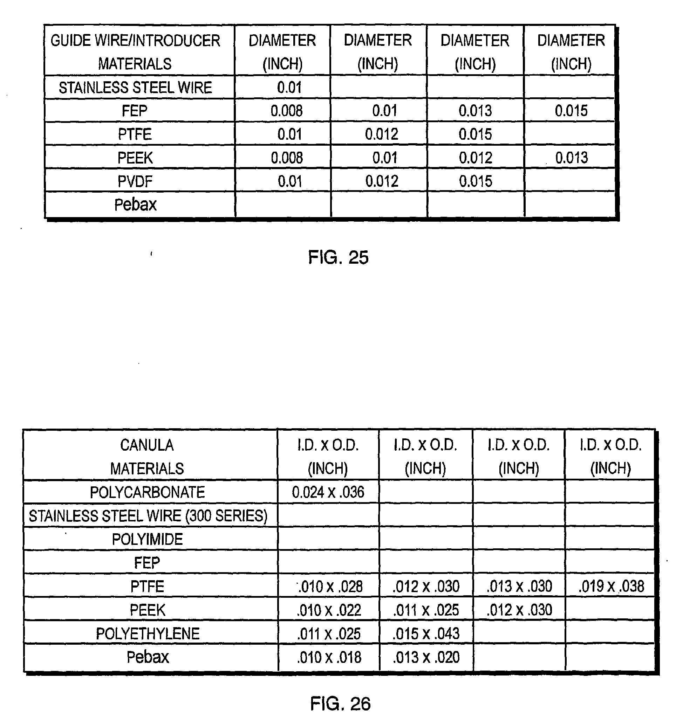

Figure 25 is a table illustrating exemplary parametric values and materials for guide wire and introducers;

Figure 26 is a table illustrating exemplary parametric values and materials for cannula; and

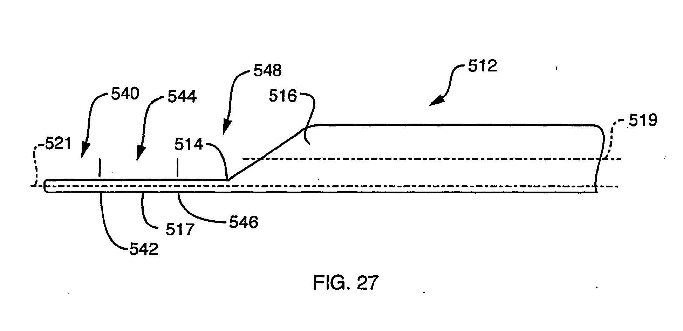

Figure 27 is a partial schematic side view of a catheter.

### DETAILED DESCRIPTION

Figures 15-17 illustrate an access device 10 for accessing a body orifice, such as a breast duct. The access device 10 comprises a catheter 12 having a distal end 14 with a centrally disposed single lumen 16 which extends the length of the catheter 12, and an elongated distal tip portion 17 which extends beyond the distal end 14 a predetermined distance. The center axis 21 of distal tip portion 17 extends substantially parallel to the center axis 19 of single lumen 16 and is disposed within the wall of the catheter. In such an exemplary construction, the distal tip portion 17 is disposed "off-axis" or "off-center" from center axis 19 of lumen 16. The off-axis construction of distal tip portion 17 advantageously maintains the open inner diameter of the catheter for maximum fluid flow in and out of the breast duct. Further, the off axis configuration allows substantially improved flow because it does not to interfere with the introduction or removal of fluid and collected samples so not to compromise the ability to retrieve a meaningful sample while still being in direction of the catheter and lumen. The elongated configuration of the distal tip portion 17 acts as an effective guide while not interfering with fluid infusion or compromising ductal fluid sample collection. Distal tip portion 17 also provides ease of insertion of catheter 12 in the ductal orifice. In one construction, distal tip portion 17 is flexible in nature so as to allow the catheter to traverse the potentially tortuous and/or angled geometry of the human breast duct. Further, distal tip portion 17 distends the ductal orifice to reduce the associated pain upon insertion of the catheter therein.

Catheter 12 has dimensions which permit introduction of the distal end 14 through a ductal orifice and positioning a distal end thereof distal to the ductal sphincter of a human breast. In one construction, the catheter 12 has a maximum outer diameter of approximately 1 mm (0.039 inches) so as to cannulate the ductal orifices of the breast. Nevertheless, other dimensions are possible for the outer diameter (e.g., approximately 0.64 mm to 1 mm (0.025 to 0.039 inches)). Single lumen 16 with an internal ID of approximately 0.64 mm (0.025 inches) accesses the breast duct and through which fluid is infused, and from which ductal fluid samples including ductal epithelial cells are collected or drawn up out of the duct. Distal tip portion 17 has dimensions which permit introduction through a ductal orifice so as to lead the catheter 12 into the breast duct. In one exemplary construction, distal tip portion 17 has a smaller diameter than the catheter 12 to allow ease, of insertion into the breast duct. Distal tip portion 17 may have a diameter of approximately 0.25 mm (0.010 inches) although, other dimensions are possible (e.g., 0.20 mm to 0.30 mm (0.008 to 0.012 inches)). In an alternative construction, the distal tip portion 17 may be flexible.

Referring to Figures 16 and 17, the distal tip portion 17 may have a tapered configuration being largest at the distal end 14 of the lumen 16 (i.e., proximal end of distal tip 17) extending therefrom to be smallest at the distal end 18. In use, the taper and flexibility of the distal tip portion 17 guides the catheter for intraductal movement. Shown in Figure 17, the transition between the distal tip portion 17 and catheter 12 includes a beveled surface 23. This beveled surface 23 provides a smooth transition between distal tip portion 17 of catheter 12 which makes the catheter 12 easy to introduce into the ductal opening after the insertion of the tip portion 17 into the breast duct. Hence, the distal tip portion 17 may hold the duct opening in position so that the catheter 12 enters with relative ease. As may be appreciated by one of ordinary skill in the art, the beveled surface 23 reduces the stress level on the tissue being penetrated and spread open by the distal end 14 of the catheter 12. In contrast to traditional catheters, discomfort to the patient is greatly reduced with the access device 10 of the present invention. The edges of the catheter at the distal end and the end 18 of the distal tip 17 may include an atraumatic configuration. In one atraumatic configuration, the edges and end 18 are rounded to reduce friction and eliminate surfaces that could puncture or otherwise injure the duct. Thus, this construction reduces localized trauma to the tissue verses non-atraumatic designs.

Referring to Figure 15, a multiport hub 22 is coupled to the proximal end 15 of catheter 12. In a preferred construction, hub 22 includes transparent material so that the user may visualize the flow to and from the breast duct during a lavage procedure. The hub 22 has a low profile so as to reduce the torque on the breast nipple after insertion of catheter 12. This overcomes the excessive generated torque on the breast nipple known to cause obstruction of ductal fluid due to compression of the tissue. Thus, improved collection of ductal cellular material is provided.

Hub 22 is attached to a tubing set 25 that comprises an infusion tube 24 from which fluid is infused into lumen 16 through hub 22 and a collection tube 26 from which fluid is collected from lumen 16 via hub 22. Infusion tube 24 and collection tube 26 are attached to hub 22 in a conventional manner to allow fluid flow. In one construction, infusion tube 24 and collection tube 26 are translucent and have standard luer connections at their distal ends that mate with ports on the hub 22 and receive fluids. The proximal ends of the tubes 24, 26 also include standard luer connection that a practitioner or attendant to manage the various ductal fluids using an appropriate syringe with a standard male luer. If desired, tubes 24, 26 may be labeled to indicate the inflow and outflow paths, e.g. infusion or collection functions.

In a further construction, an optional pinch clamp or other flow control device (not shown) may be disposed on the outflow tube, collection tube 26. In use, the clamp closes the collection tube 24 to prevent fluid leakage from the tubing during fluid infusion into a cannulated breast duct. In one construction, hub 22 includes an ergonomic handle 27 for the user to grasp during a ductal lavage procedure. It should be recognized that a fluid used in the hub 22 and catheter 12 may be virtually any appropriate fluid for the ductal lavage procedure. Exemplary ductal wash fluids which may be used with hub 22 includes but is not limited to saline, phosphate buffered saline, a nonabsorbable fluid, an isotonic solution, an osmotic solution, a hypotonic solution, and a hypertonic solution. Nevertheless, an appropriate therapeutic fluid may be provided by way of the ductal access devices described herein. Alternatively, the fluids could include diagnostic or therapeutic fluids carrying diagnostic and/or therapeutic agents.

In an alternative construction depicted in Figures 19 and 20, an inventive ductal access system 100, includes a catheter 112 having a distal end 114 with a centrally disposed single lumen 116 which extends the length of the catheter 112. Catheter 112 includes a tubular introducer pathway 120 adapted to slideably receive a ductal introducer 118 therein. Long axis 121 of pathway 120 is offset but substantially parallel to long axis 119 of lumen 116. For ease of explanation, as used herein the term "introducer" is defined to include guidewires, dilators, stylettes or portion of any of these that may be inserted within a passageway of a catheter or into the ductal orifice. Ductal introducer 118 is provided for ease of varying the length of tip portion 117 so as to improve intraductal travel of the catheter 112 within the breast duct and to reduce risk of puncturing the ductal wall and rupturing the breast duct. By removably inserting the ductal introducer 118 into the axis 121 of pathway 120, the user is able to choose an introducer having a desired stiffness for the particular patient. Additionally, this enables the practitioner or attendant to change the flexibility and stiffness of the introducer 118. The stiffness and flexibility may be a function of a material property and/or cross-sectional shape. One example of a material property that relates to flexibility is the modulus of elasticity. In another example, the cross-sectional shape of the introducer 118 may be circular, elliptical, oval or other shapes that provide predetermined stiffness in one or more directions. One of skill in the art would recognize these various shapes relate to a section modulus of the introducer 118. Therefore, it should be recognized that introducer pathway 120 may be virtually any appropriate cross-sectional shape to meet the cross-sectional shape of the introducer 118.

Ductal introducer 118 includes a distal end 122, which enters the breast duct. The opposing proximal end 124 of ductal introducer 118 includes a handle 126. Handle 126 provides ease of operation so that a user may grasp and manipulate the introducer 118 to access and navigate the breast duct anatomy. Ductal access device 100 includes a hub 22 which has similar construction and components as hub 22 shown in Figure 15. In hub 22, the introducer pathway 120 extends through so that the introducer 118 may be inserted. The pathway may be a tube extending through the exterior of the hub 22 or the tube may be located on the outer surface of the hub. An adjustable tip portion 117 of introducer 118 is defined from the distal end 122 of ductal introducer 118 to the distal end portion 114 of catheter 112. In this configuration, the length of the tip portion 117 is selectively adjustable by the user for accessing and traversing the breast duct. In one aspect, ductal introducer 118 may be embodied as a guide wire which is easily inserted into pathway 120 and into the breast duct. In one construction shown in Figure 20, the center axis 121 of introducer pathway 120 extends substantially parallel to the center axis 119 of single lumen 116. As discussed above, in such an exemplary construction, the introducer pathway 120 is disposed "off-axis" or "off-center" from center axis 119 of lumen 116. Thus, ductal introducer 118 with the off-axis construction of catheter 112 provides similar benefits as the ductal access device 10.

The introducers described herein may be formed of appropriate cross-sectional shapes and various materials for medical use. The shapes and materials enable desired rigidity and flexibility for intraductal movement. Alternative materials for the introducer 118 may include but are not limited to: Stainless Steel Wire; FEP; PTFE; PEEK; and PVDF and PEBAX. Nevertheless, other appropriate materials may be employed. Specific dimensional value introducers are shown in Figure 25, but are provided by way of example. Alternate coatings for the introducer and/or catheter may include but are not limited to: hydromer coating; STS SLIP-COAT; MDX; silicone dry; silicone lubricant; PTFE coatings. The specific thickness of the coatings and application may be readily determined by one of ordinary skill in the art.

The introducer may be made of metal or plastics and may have a tapered and/or an atraumatic tip for gently probing and accessing a breast duct. In one example, the introducer may be constructed of a superelastic or shape memory material. As used herein, the term "superelastic shape memory material" refers to a class of metal alloys that have a stress-induced phase change or temperate from austenite to martensite and upon stress release, the material springs back to this original phase and shape. The material structure of a superelastic shape memory material regarding austenite and martensite is well-known to one of ordinary skill in the metallurgy art. A NiTi material or NiTi alloy may be used as an alloy material for the introducer. As used herein, a NiTi superelastic shape memory material refers to an alloy that is an intermetallic compound of nickel and titanium having nearly equal mixtures as measured by weight. One composition of a NiTi superelastic shape memory material generally has a greater percentage of nickel by weight than titanium, such as 51%-56% of nickel, and preferably 54-55% nickel. The specific percentages of nickel and titanium may be adjusted by one of ordinary skill in the art.

It is not necessary for the introducer to be composed of a material that has shape memory characteristics. The introducer may also be formed of a stiff material such as a metal wire or a flexible material such as plastic. In an embodiment of the invention, the combined introducer may be formed of Nitinol due to its flexibility, durability, and biocompatibility. In an alternative embodiment, the introducer may be formed of multiple materials or the same materials having different stiffnesses. As a result, the introducer may have sections that are more flexible than adjacent sections of the same introducer. As a result, for example, the introducer may have a first, stiff portion for guiding the introducer within the ductal lumen and a second, flexible portion that allows the introducer to conform to the shape of the ductal lumen or lumen branch into which it is introduced. In any of the above-discussed embodiments, the introducer may be coated with a liquid or dry lubricant material that reduces the friction between the introducer and the breast duct during the introduction and advancement of the introducer in the duct.

Figures 21 and 22 illustrate an alternative construction of a catheter body 212 having a distal end 214 with a centrally (about axis 219) disposed single lumen 216 that traverses the length of the catheter body. In an off-axis configuration, an elongated flexible tip 217 extends beyond the distal end 214 along axis 221. The tip 217 is configured to receive a removable introducer 218. This feature is achieved in that the tip 217 and catheter body 212 include an internal passageway 220 adapted to slideably receive the introducer 218. The passageway 220 may be tubular at the tip 217 and is located in the walls of catheter body 212. Passageway 220 has a closed distal end 222, which is in the initial portion that enters the breast duct. The passageway 220 is opened at the proximal end. The closed end feature enables the practitioner or user to adjust the flexibility of the tip 217 during cannulation of the breast duct but prevent fluid from entering the passageway 220. Further, the closed end feature allows the introducer 218 to be removed and another introducer inserted therein when the catheter 212 is positioned within the breast duct. Thus, introducer 218 may be removed or inserted into the tip 217 to selectively adjust the flexibility of tip 217. This allows the user to insert the introducer 218 making the flexible tip more rigid for insertion into the duct, or remove the stylette making the tip more flexible so the tip may transverse tortuous ductal geometry. In this configuration, passageway 220 extends the length of the lumen 216 and is parallel thereto. Advantageously, an aspect of the present invention provides an option to change to tip flexibility which greatly aids the cannulation of the breast duct. The material and thickness of flexible tip 217 may be chosen to achieve a desired stiffness in combination with the flexure characteristics of introducer 218. The closed distal end 222 is rounded to reduce friction between the ductal tissue and tip 217 and prevent the distal end 222 from puncturing duct.

Figure 22A illustrates an alternative catheter body 312, which has similar construction as catheter body 212 except that a passageway 320 is in the distal tip 317 and not in the walls of the catheter body 312. This allows the user to insert the introducer 218 through the lumen 316 and into the proximal end 311 of the passageway 320. Flexible elongated distal tip 317 includes a closed distal end 322 extending to the distal end 314 of catheter body 312.

As shown in Figures 22 and 22A, the catheter 212, 312 may be provided with a plurality of introducers each of which have a different stiffness characteristic or property. Introducers are provided depending on the portion of duct being accessed. One introducer may be used to introduce the distal tip 217, 317 past the ductal sphincter. Another introducer may be provided to enter a desired branch of breast duct. Yet another introducer, if desired, may be used to enter a final portion of branch. This allows practitioner to design stiffness of tip 217, 317 to match needs presented by different portions of the duct.

An introducer may be designated with a stiffness value to provide a certain stiffness property relative to the other introducers. Purely by way of example, a practitioner may be provided with an introducer having a stiffness value of two which is designed to be rigid so as to allow penetrating the ductal opening. Another introducer may be provided having a stiffness value four which would be less stiff than a value of two. Yet another introducer may have a value of eight which would be less stiff than a value of four. In this example, a practitioner is enabled to access the breast duct with a rigid introducer inserted within the passageway 220, 320. The practitioner, while the catheter 212, 312 resides within the breast duct, may remove the rigid introducer and an insert another introducer to continue to advance within the breast duct. Thus, the practitioner is allowed to enter the breast with the catheters 212, 312 without removing the catheter once the breast duct is accessed. Advantageously, catheters 212, 312 allow the practitioner to be more efficient in the ductal access procedure because steps are eliminated or substantially reduced over conventional procedures, e.g., the use of dilators be significantly reduced. Further, patient comfort is increased because less traumatic movement is provided to the breast.

In another construction shown in Figure 18, a catheter 412 has a distal end 414 with a single lumen 416 disposed about a central axis 419 which extends the length of the catheter, and an elongated distal tip portion 417 which extends beyond the distal end 414. Catheter 412 has a bevel face and the tip portion 417 includes an extensible introducer 418 formed as a stylette portion. Stylette portion 418 comprises a distal end 422 extending from a distal opening 423 of a transition portion 425 of the lumen 416. Transition portion 425 includes a passageway configured to receive a stylette so that the distal end 422 exits the opening 423. The stylette portion 418 may be made of a flexible metal, plastic material or other materials described above. The stylette portion 418 may be fixed in position and serve as a flexible tip, or it may be removed after insertion of the catheter into the duct. The stylette portion 418 may be removed and replaced with a shorter stylette portion so as to shorten the tip length. Alternatively, the stylette portion 418 may have a longer length. In this manner, the tip length may be adjusted to accommodate the particular duct geometry.

Figure 27 illustrates an alternative construction of a catheter body 512 having a distal end 514 with a centrally (about axis 519) disposed single lumen 516 that traverses the length of the catheter body. In an off-axis configuration, an elongated tip 517 extend beyond the distal end 514 along axis 521. The elongated tip 517 may have a multi-flexion configuration that has separate regions of different flexions that each corresponds to the flexibility, or lack thereof, needed to access a respective portion of a breast duct. This multi-flexion zone configuration provides adaptability for a practitioner to reduce steps for accessing a breast and/or customize the access of the duct to increase patient comfort. In one exemplary example, the elongated tip 517 may have three flex zones to accommodate to access a breast duct. A first flexion zone 540 may extend from the distal end to a first intermediate position 542 along the length of the elongated tip. The first flexion zone 540 maybe substantially rigid for entering into the breast duct to overcome the resistance force provided by the tissue at the ductal opening. An adjacent second flexion zone 544 may extend to another intermediate position away from first intermediate position 542 along the length of the elongated tip 517. The second flexion zone 544 may be less rigid than a first flex zone 544 so as to allow the elongated tip 517 to traverse the breast duct geometry. A third flex zone 548 may be provided adjacent to the second flexion zone 544. The third flexion zone 548 may be more flexible than the second flexion zone. In a specific example, the dimensions of the first flexion zone maybe 3 cm from the distal end; second flexion zone may have a length of 4 to 8 cm; and the third flexion own may have a length of 2 to 3 cm. Nevertheless, the length of the zones maybe configured as desired by the practitioner. "

Figures 23A-23C are schematic diagrams illustrating a method of accessing a breast duct by way of a catheter 12 of Figure 16. Referring to Figure 23A, the catheter 12 is prepared to be introduced into a ductal orifice so that a distal end thereof will be positioned within the duct beyond the ductal sphincter during ductal lavage, introduction of diagnostic materials and/or introduction of therapeutic materials. As shown in Figure 23B, the distal end 14 of the catheter is outside of the duct. The catheter has a flexible distal tip 17 which provides ease of insertion of catheter 12 in the ductal orifice. Further, the flexible distal tip 17 is inserted into the duct so as to follow the intraductal geometry but not cause damage to the duct. As shown in Figure 23C, the distal end 14 has entered the duct. The distal tip 17 continues to follow the intraductal geometry to guide the catheter 12 there through. Further, the catheter 12 is more rigid than the distal tip 17 so as to straighten the duct for infusion and collection. Although catheter 12 is described with reference to Figure 23A-23C, other catheters 112, 212, 312, 412, and 512 may be used in a similar fashion.

Figures 24A-24C are schematic diagrams illustrating a method of accessing a breast duct with a catheter body 212 of Figure 21. Referring to Figure 24A, the catheter body 212 is prepared to be introduced into a ductal orifice. The practitioner may choose an introducer 218 of a desired flexibility or rigidity. For example, the introducer 218 of different rigidity properties may be inserted for adjusting to a desired tip characteristic during insertion of the distal tip 217 into the breast duct. This arrangement creates a composite insertion member for accessing the breast duct. The desired tip characteristic may relate to the stiffness and flexibility, which may be functions of a material property or a cross-sectional shape. One of skill in the art would recognize that the composite insertion member may have various shapes and material properties to vary the section modulus.

In particular, the tip 217 may be selectively made rigid to penetrate the duct orifice. In this configuration, the length of the catheter 212 and distal tip 217 is relatively rigid. In Figure 24B, the introducer 218, which is rigid or somewhat rigid, has been removed from the distal tip 217 while the catheter 212 is inserted in the duct. In this removed configuration, the catheter 212 may flex and bend through the intraductal geometry. If desired, an introducer 218, which is less stiff, may be inserted into the distal tip 217. With or without an introducer 218, the catheter 212 is advanced further into the duct. In Figure 24C, once a desired position is reached within the duct, a rigid or somewhat rigid introducer 218 may be inserted and advanced into the catheter 212 to straighten the duct for infusion and collection. In a shape memory embodiment, the stiffness or flexibility of introducer 218 may be adjusted in response to the addition of stimulus or removal of a stimulus without removal from the catheter 212. While not shown, it should be recognized that distal tip 217 could be inserted into the ductal orifice without the need of an introducer 218. The introducer of an appropriate stiffness may be inserted into the distal tip 217 during or after intraductal entry of the catheter 212. Although catheter 212 is described with reference to Figure 24A-24C, other catheters 112, 312, 412 and 512 may be used in a similar fashion.

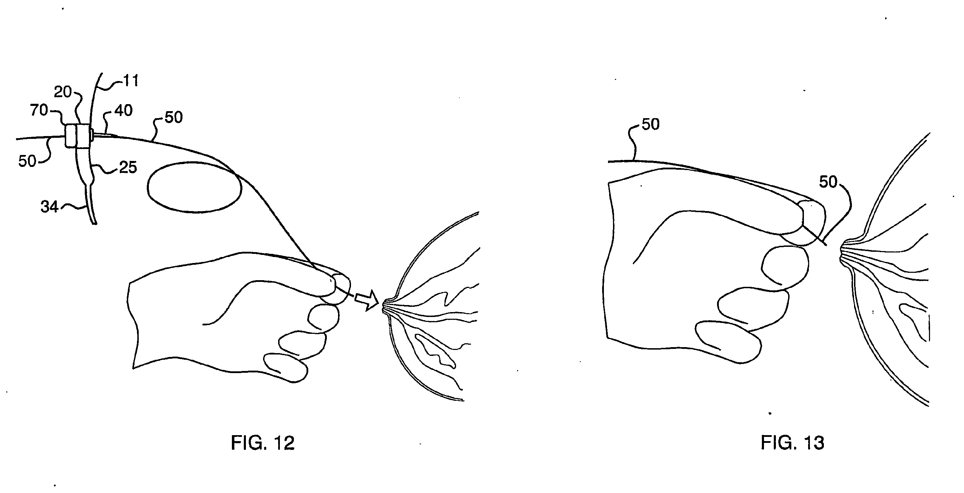

The catheters as described herein may be constructed of a wide range of appropriate materials for medical use. In a preferred construction, the catheter is formed with PTFE and coated with STS Slipcoat to reduce friction on the device during placement in the breast duct. In lieu of PTFE, alternate materials for the catheter may include but are not limited to: polycarbonate; stainless steel (300 Series); polymide; FEP; PEEK; polyethylene; and PEBAX. Specific dimensional values of catheters are shown in Figure 12, but are provided by way of example. Nevertheless, other appropriate materials and dimensional values may be employed.

The hub 22 housing may be molded from a polycarbonate. The tubing set 25 may be made of PVC with luer connectors made of polycarbonate. The tubing set may be made of a variety of conventional materials including but not limited to: silicone; low density polyethylene; PVC; tygon; or polypropylene.

In one manufacturing process, the catheters according to one or more aspects of the present invention may be constructed by using an extrusion process. After the process, the distal end of catheter is shaped by molding and/or cutting. An optional, bevel may be is cut to provide the transition between the distal tip portion and the catheter. The edges of the catheter may be rounded using an RF tipping process known to one of ordinary skill in the art. The catheter may be bonded to the hub using a desired medical grade UV adhesive. In turn, the tubes may be bonded to the hub and to the luer connector using UV adhesive as well. If desired, a pinch clamp may be placed on the outflow tube.

The device of the present invention may be used in a method for obtaining cellular material from a human breast milk duct includes introducing a ductal access device such as devices 12, 112, 212, 312, 412, and 512 having at least one lumen into a duct. A wash fluid may be introduced through the access device internal lumen into the milk duct. In the method, a volume of at least 2 ml of wash fluid may be present within the duct for a preselected time, and then at least a portion of the wash fluid is collected from the duct through the lumen of the access device. The method may further comprise massaging and squeezing the breast tissue after introducing the wash fluid but prior to and/or during collecting a portion of the wash fluid. Introducing the ductal access device typically comprises positioning a distal end of the catheter distal to the ductal sphincter in the breast duct. The access device may include only a single lumen that extends into the duct. The preselected time may be less than one second, but will usually be in the range from one second to one hour.

### DUCTAL LAVAGE CATHETER

The device of the present invention may also comprise an introducer having different lengths. The introducer may extend at least approximately 6.35 mm, 12.50 mm or 24.99 mm (0.250, 0.492, or 0.984 inches) beyond the distal tip of the catheter. The distal tip of the catheter may have an atraumatic configuration.

### BRIEF DESCRIPTION OF THE DRAWINGS

Figure 15 is a schematic representation of a first ductal access device in accordance with one or more aspects of the present invention;

Figure 16 is a perspective view of a catheter;

Figure 17 is a partial schematic side view of a catheter;

Figure 18 is a schematic side view of a catheter with a removable tip;

Figure 19 is a schematic representation of a second ductal access device in accordance the present invention

Figure 20 is a sectional view of a catheter shown in FIG. 19 taken along an central axis to illustrate an interior construction and components;

Figure 21 is a perspective view of a catheter;

Figure 22 is a sectional view of a catheter shown in FIG. 21 taken along an central axis to illustrate an interior construction and components;

Figure 22A is a sectional view similar to FIG. 22 showing an alterative embodiment of a catheter;

Figures 23A-24C are diagrams illustrating a method of accessing a breast duct which a catheter of FIG. 16;

Figures 24A-24C are diagrams illustrating a method of accessing a breast duct which a catheter of FIG. 21;

Figure 25 is a table illustrating exemplary parametric values and materials for guide wire and introducers;

Figure 26 is a table illustrating exemplary parametric values and materials for cannula; and

Figure 27 is a partial schematic side view of a catheter.

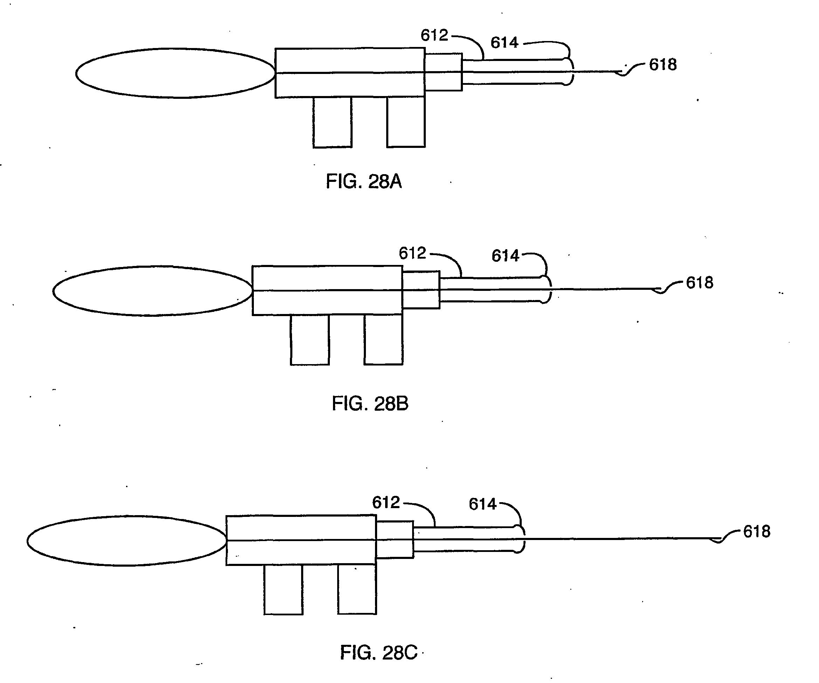

Figure 28 is a schematic representation of an alternative ductal access device that does not form part of the present invention.

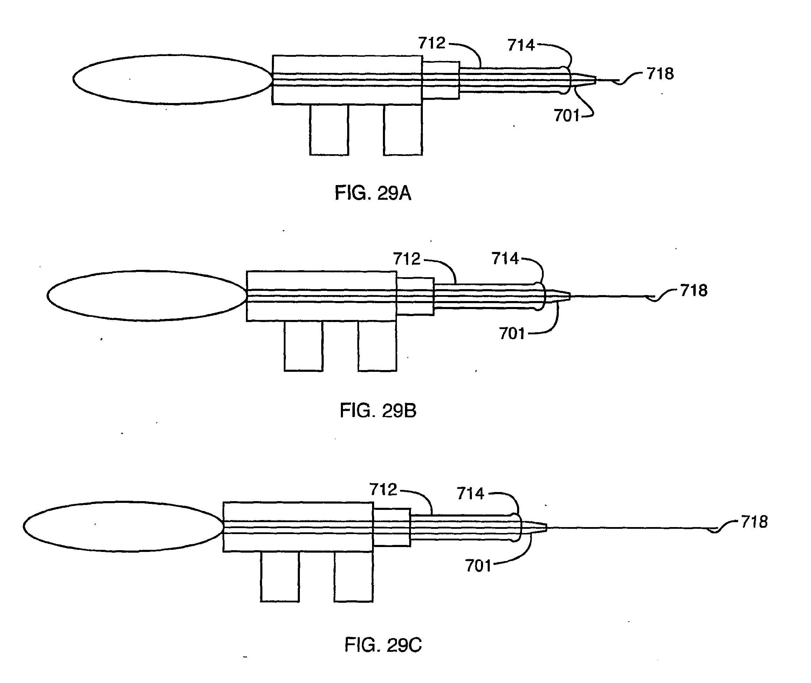

Figure 29 is a schematic representation of an alternative ductal access device that does not form part of the present invention.

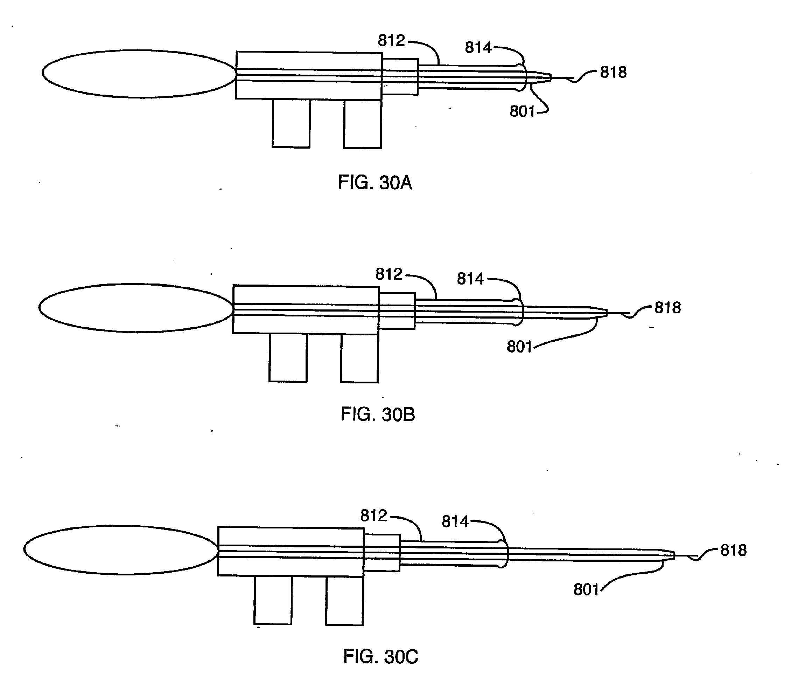

Figure 30 is a schematic representation of an alternative ductal access device in that does not form part of the present invention.

### DETAILED DESCRIPTION

Figures 15-17 illustrate an access device 10 for accessing a body orifice, such as a breast duct. The access device 10 comprises a catheter 12 having a distal end 14 with a centrally disposed single lumen 16 which extends the length of the catheter 12, and an elongated distal tip portion 17 which extends beyond the distal end 14 a predetermined distance. The center axis 21 of distal tip portion 17 extends substantially parallel to the center axis 19 of single lumen 16 and is disposed within the wall of the catheter. In such an exemplary construction, the distal tip portion 17 is disposed "off-axis" or "off-center" from center axis 19 of lumen 16. The off-axis construction of distal tip portion 17 advantageously maintains the open inner diameter of the catheter for maximum fluid flow in and out of the breast duct. Further, the off axis configuration allows substantially improved flow because it does not to interfere with the introduction or removal of fluid and collected samples so not to compromise the ability to retrieve a meaningful sample while still being in direction of the catheter and lumen. The elongated configuration of the distal tip portion 17 acts as an effective guide while not interfering with fluid infusion or compromising ductal fluid sample collection. Distal tip portion 17 also provides ease of insertion of catheter 12 in the ductal orifice. In one construction, distal tip portion 17 is flexible in nature so as to allow the catheter to traverse the potentially tortuous and/or angled geometry of the human breast duct. Further, distal tip portion 17 distends the ductal orifice to reduce the associated pain upon insertion of the catheter therein.

Catheter 12 has dimensions with permit introduction of the distal end 14 through a ductal orifice and positioning a distal end thereof distal to the ductal sphincter of a human breast. In one construction, the catheter 12 has a maximum outer diameter of approximately 1 mm (0.039 inches) so as to cannulate the ductal orifices of the breast. Nevertheless, other dimensions are possible for the outer diameter (e.g., approximately 0.64 mm to 1 mm (0.025 to 0.039 inches)). Single lumen 16 with an internal ID of approximately 0.64 mm (0.025 inches) accesses the breast duct and through which fluid is infused, and from which ductal fluid samples including ductal epithelial cells are collected or drawn up out of the duct. Distal tip portion 17 has dimensions which permit introduction through a ductal orifice so as to lead the catheter 12 into the breast duct In one exemplary construction, distal tip portion 17 has a smaller diameter than the catheter 12 to allow ease, of insertion into the breast duct. Distal tip portion 17 may have a diameter of approximately 0.254 mm (0.010 inches), although other dimensions are possible (e.g., 0.20 mm to 0.30 mm (0.008 to 0.012 inches)). In an alternative construction, the distal tip portion 17 may be flexible.

Referring to Figures 16 and 17, the distal tip portion 17 may have a tapered configuration being largest at the distal end 14 of the lumen 16 (i.e., proximal end of distal tip 17) extending therefrom to be smallest at the distal end 18. In use, the taper and flexibility of the distal tip portion 17 guides the catheter for intraductal movement. Shown in Figure 17, the transition between the distal tip portion 17 and catheter 12 includes a beveled surface 23. This beveled surface 23 provides a smooth transition between distal tip portion 17 of catheter 12, which makes the catheter 12 easy to introduce into the ductal opening after the insertion of the tip portion 17 into the breast duct Hence, the distal tip portion 17 may hold the duct opening in position so that the catheter 12 enters with relative ease. As may be appreciated by one of ordinary skill in the art, the beveled surface 23 reduces the stress level on the tissue being penetrated and spread open by the distal end 14 of the catheter 12. In contrast to traditional catheters, discomfort to the patient is greatly reduced with the access device 10 of the present invention. The edges of the catheter at the distal end and the end 18 of the distal tip 17 may include an atraumatic configuration. In one atraumatic configuration, the edges and end 18 are rounded to reduce friction and eliminate surfaces that could puncture or otherwise injure the duct Thus, this construction reduces localized trauma to the tissue verses non-atraumatic designs.

Referring to Figure 15, a multiport hub 22 is coupled to the proximal end 15 of catheter 12. In a preferred construction, hub 22 includes transparent material so that the user may visualize the flow to and from the breast duct during a lavage procedure. The hub 22 has a low profile so as to reduce the torque on the breast nipple after insertion of catheter 12. This overcomes the excessive generated torque on the breast nipple known to cause obstruction of ductal fluid due to compression of the tissue. Thus, improved collection of ductal cellular material is provided.

Hub 22 is attached to a tubing set 25 which comprises an infusion tube 24 from which fluid is infused into lumen 16 through hub 22 and a collection tube 26 from which fluid is collected from lumen 16 via hub 22. Infusion tube 24 and collection tube 26 are attached to hub 22 in a conventional manner to allow fluid flow. In one construction, infusion tube 24 and collection tube 26 are translucent and have standard luer connections at their distal ends that mate with ports on the hub 22 and receive fluids. The proximal ends of the tubes 24, 26 also include standard luer connection that a practitioner or attendant to manage the various ductal fluids using an appropriate syringe with a standard male luer. If desired, tubes 24, 26 may be labeled to indicate the inflow and outflow paths, e.g. infusion or collection functions.

In a further construction, an optional pinch clamp or other flow control device (not shown) may be disposed on the outflow tube, collection tube 26. In use, the clamp closes the collection tube 24 to prevent fluid leakage from the tubing during fluid infusion into a cannulated breast duct. In one construction, hub 22 includes an ergonomic handle 27 for the user to grasp during a ductal lavage procedure. It should be recognized that a fluid used in the hub 22 and catheter 12 may be virtually any appropriate fluid for the ductal lavage procedure. Exemplary ductal wash fluids which may be used with hub 22 includes but is not limited to saline, phosphate buffered saline, a nonabsorbable fluid, an isotonic solution, an osmotic solution, a hypotonic solution, and a hypertonic solution. Nevertheless, an appropriate therapeutic fluid may be provided by way of the ductal access devices described herein. Alternatively, the fluids could include diagnostic or therapeutic fluids carrying diagnostic and/or therapeutic agents.

In an alternative construction depicted in Figures 19 and 20, an inventive ductal access system 100, includes a catheter 112 having a distal end 114 with a centrally disposed single lumen 116 which extends the length of the catheter 112. Catheter 112 includes a tubular introducer pathway 120 adapted to slideably receive a ductal introducer 118 therein. Long axis 121 of pathway 120 is offset but substantially parallel to long axis 119 of lumen 116. For ease of explanation, as used herein the term "introducer" is defined to include guidewires, dilators, stylettes or portion of any of these that may be inserted within a passageway of a catheter or into the ductal orifice. Ductal introducer 118 is provided for ease of varying the length of tip portion 117 so as to improve intraductal travel of the catheter 112 within the breast duct and to reduce risk of puncturing the ductal wall and rupturing the breast duct. By removably inserting the ductal introducer 118 into the axis 121 of pathway 120, the user is able to choose an introducer having a desired stiffness for the particular patient. Additionally, this enables the practitioner or attendant to change the flexibility and stiffness of the introducer 118. The stiffness and flexibility may be a function of a material property and/or cross-sectional shape. One example of a material property that relates to flexibility is the modulus of elasticity. In another example, the cross-sectional shape of the introducer 118 may be circular, elliptical, oval or other shapes that provide predetermined stiffness in one or more directions. One of skill in the art would recognize these various shapes relate to a section modulus of the introducer 118. Therefore, it should be recognized that introducer pathway 120 may be virtually any appropriate cross-sectional shape to meet the cross-sectional shape of the introducer 118.

Ductal introducer 118 includes a distal end 122, which enters the breast duct. The opposing proximal end 124 of ductal introducer 118 includes a handle 126. Handle 126 provides ease of operation so that a user may grasp and manipulate the introducer 118 to access and navigate the breast duct anatomy. Ductal access device 100 includes a hub 22 which has similar construction and components as hub 22 shown in Figure 15. In hub 22, the introducer pathway 120 extends through so that the introducer 118 may be inserted. The pathway may be a tube extending through the exterior of the hub 22 or the tube may be located on the outer surface of the hub. An adjustable tip portion 117 of introducer 118 is defined from the distal end 122 of ductal introducer 118 to the distal end portion 114 of catheter 112. In this configuration, the length of the tip portion 117 is selectively adjustable by the user for accessing and traversing the breast duct. In one aspect, ductal introducer 118 may be embodied as a guide wire, which is easily inserted into pathway 120 and into the breast duct. In one construction shown in Figure 20, the center axis 121 of introducer pathway 120 extends substantially parallel to the center axis 119 of single lumen 116. As discussed above, in such an exemplary construction, the introducer pathway 120 is disposed "off-axis" or "off-center" from center axis 119 of lumen 116. Thus, ductal introducer 118 with the off-axis construction of catheter 112 provides similar benefits as the ductal access device 10.

The introducers described herein may be formed of appropriate cross-sectional shapes and various materials for medical use. The shapes and materials enable desired rigidity and flexibility for intraductal movement. Alternative materials for the introducer 118 may include but are not limited to: Stainless Steel Wire; FEP; PTFE; PEEK; and PVDF and PEBAX. Nevertheless, other appropriate materials may be employed. Specific dimensional value introducers are shown in Figure 25, but are provided by way of example. Alternate coatings for the introducer and/or catheter may include but are not limited to: hydromer coating; STS SLIP-COAT; MDX; silicone dry; silicone lubricant; PTFE coatings. The specific thickness of the coatings and application may be readily determined by one of ordinary skill in the art.

The introducer may be made of metal or plastics and may have a tapered and/or an atraumatic tip for gently probing and accessing a breast duct. In one example, the introducer may be constructed of a superelastic or shape memory material. As used herein, the term "superelastic shape memory material" refers to a class of metal alloys that have a stress-induced phase change or temperate from austenite to martensite and upon stress release, the material springs back to this original phase and shape. The material structure of a superelastic shape memory material regarding austenite and martensite is well known to one of ordinary skill in the metallurgy art. A NiTi material or NiTi alloy may be used as an alloy material for the introducer. As used herein, a NiTi superelastic shape memory material refers to an alloy that is an intermetallic compound of nickel and titanium having nearly equal mixtures as measured by weight. One composition of a NiTi superelastic shape memory material generally has a greater percentage of nickel by weight than titanium, such as 51%-56% of nickel, and preferably 54-55% nickel. The specific percentages of nickel and titanium may be adjusted by one of ordinary skill in the art.

It is not necessary for the introducer to be composed of a material that has shape memory characteristics. The introducer may also be formed of a stiff material such as a metal wire or a flexible material such as plastic. In an embodiment of the invention, the combined introducer may be formed of Nitinol due to its flexibility, durability, and biocompatibility. In art alternative embodiment, the introducer may be formed of multiple materials or the same materials having different stiffnesses. As a result, the introducer may have sections that are more flexible than adjacent sections of the same introducer. As a result, for example, the introducer may have a first, stiff portion for guiding the introducer within the ductal lumpen and a second, flexible portion that allows the introducer to conform to the shape of the ductal lumen or lumen branch into which it is introduced. In any of the above-discussed embodiments, the introducer may be coated with a liquid or dry lubricant material that reduces the friction between the introducer and the breast duct during the introduction and advancement of the introducer in the duct.

Figures 21 and 22 illustrate an alternative construction of a catheter body 212 having a distal end 214 with a centrally (about axis 219) disposed single lumen 216 that traverses the length of the catheter body. In an off-axis configuration, an elongated flexible tip 217 extends beyond the distal end 214 along axis 221. The tip 217 is configured to receive a removable introducer 218. This feature is achieved in that the tip 217 and catheter body 212 include an internal passageway 220 adapted to slideably receive the introducer 218. The passageway 220 may be tubular at the tip 217 and is located in the walls of catheter body 212. Passageway 220 has a closed distal end 222, which is in the initial portion that enters the breast duct. The passageway 220 is opened at the proximal end. The closed end feature enables the practitioner or user to adjust the flexibility of the tip 217 during cannulation of the breast duct but prevent fluid from entering the passageway 220. Further, the closed end feature allows the introducer 218 to be removed and another introducer inserted therein when the catheter 212 is positioned within the breast duct. Thus, introducer 218 may be removed or inserted into the tip 217 to selectively adjust the flexibility of tip 217. This allows the user to insert the introducer 218 making the flexible tip more rigid for insertion into the duct, or remove the stylette making the tip more flexible so the tip may transverse tortuous ductal geometry. In this configuration, passageway 220 extends the length of the lumen 216 and is parallel thereto. Advantageously, an aspect of the present invention provides an option to change to tip flexibility which greatly aids the cannulation of the breast duct. The material and thickness of flexible tip 217 may be chosen to achieve a desired stiffness in combination with the flexure characteristics of introducer 218. The closed distal end 222 is rounded to reduce friction between the ductal tissue and tip 217 and prevent the distal end 222 from puncturing duct.

Figure 22A illustrates an alternative catheter body 312, which has similar construction as catheter body 212 except that a passageway 320 is in the distal tip 317 and not in the walls of the catheter body 312. This allows the user to insert the introducer 218 through the lumen 316 and into the proximal end 311 of the passageway 320. Flexible elongated distal tip 317 includes a closed distal end 322 extending to the distal end 314 of catheter body 312.

As shown in Figures 22 and 22A, the catheter 212, 312 may be provided with a plurality of introducers each of which have a different stiffness characteristic or property. Introducers are provided depending on the portion of duct being accessed. One introducer may be used to introduce the distal tip 217, 317 past the ductal sphincter. Another introducer may be provided to enter a desired branch of breast duct. Yet another introducer, if desired, may be used to enter a final portion of branch. This allows practitioner to design stiffness of tip 217, 317 to match needs presented by different portions of the duct.

An introducer may be designated with a stiffness value to provide certain a stiffness property relative to the other introducers. Purely by way of example, a practitioner may be provided with an introducer having a stiffness value of two, which is designed to be rigid so as to allow penetrating the ductal opening. Another introducer may be provided having a stiffness value four which would be less stiff than a value of two. Yet another introducer may have a value of eight, which would be less stiff than a value of four. In this example, a practitioner is enabled to access the breast duct with a rigid introducer inserted within the passageway 220, 320. The practitioner, while the catheter 212, 312 resides within the breast duct, may remove the rigid introducer and an insert another introducer to continue to advance within the breast duct. Thus, the practitioner is allowed to enter the breast with the catheters 212, 312 without removing the catheter once the breast duct is accessed. Advantageously, catheters 212, 312 allow the practitioner to be more efficient in the ductal access procedure because steps are eliminated or substantially reduced over conventional procedures, e.g., the use of dilators be significantly reduced. Further, patient comfort is increased because less traumatic movement is provided to the breast.

In another construction shown in Figure 18, a catheter 412 has a distal end 414 with a single lumen 416 disposed about a central axis 419, which extends the length of the catheter, and an elongated distal, tip portion 417, which extends beyond the distal end 414. Catheter 412 has a bevel face and the tip portion 417 includes an extensible introducer 418 formed as a stylette portion. Stylette portion 418 comprises a distal end 422 extending from a distal opening 423 of a transition portion 425 of the lumen 416. Transition portion 425 includes a passageway configured to receive a stylette so that the distal end 422 exits the opening 423. The stylette portion 418 may be made of a flexible metal, plastic material or other materials described above. The stylette portion 418 may be fixed in position and serve as a flexible tip, or it may be removed after insertion of the catheter into the duct. The stylette portion 418 may be removed and replaced with a shorter stylette portion so as to shorten the tip length. Alternatively, the stylette portion 418 may have a longer length. In this manner, the tip length may be adjusted to accommodate the particular duct geometry.

Figures 27 illustrates an alternative construction of a catheter body 512 having a distal end 514 with a centrally (about axis 519) disposed single lumen 516 that traverses the length of the catheter body. In an off-axis configuration, an elongated tip 517 extends beyond the distal end 514 along axis 521. The elongated tip 517 may have a multi-flexion configuration that has separate regions of different flexions that each corresponds to the flexibility, or lack thereof, needed to access a respective portion of a breast duct. This multi-flexion zone configuration provides adaptability for a practitioner to reduce steps for accessing a breast and/or customize the access of the duct to increase patient comfort. In one exemplary example, the elongated tip 517 may have three flex zones to accommodate to access a breast duct. A first flexion zone 540 may extend from the distal end to a first intermediate position 542 along the length of the elongated tip. The first flexion zone 540 maybe substantially rigid for entering into the breast duct to overcome the resistance force provided by the tissue at the ductal opening. An adjacent second flexion zone 544 may extend to another intermediate position away from first intermediate position 542 along the length of the elongated tip 517. The second flexion zone 544 may be less rigid than a first flex zone 544 so as to allow the elongated tip 517 to traverse the breast duct geometry. A third flex zone 548 may be provided adjacent to the second flexion zone 544. The third flexion zone 548 may be more flexible than the second flexion zone. In a specific example, the dimensions of the first flexion zone maybe 3 cm from the distal end; second flexion zone may have a length of 4 to 8 cm; and the third flexion own may have a length of 2 to 3 cm. Nevertheless, the length of the zones maybe configured as desired by the practitioner.

Figures 23A-23C are schematic diagrams illustrating a method of accessing a breast duct by way of a catheter 12 of Figure 16. Referring to Figure 23A, the catheter 12 is prepared to be introduced into a ductal orifice so that a distal end thereof will be positioned within the duct beyond the ductal sphincter during ductal lavage, introduction of diagnostic materials and/or introduction of therapeutic materials. As shown in Figure 23B, the distal end 14 of the catheter is outside of the duct. The catheter has a flexible distal tip 17, which provides ease of insertion of catheter 12 in the ductal orifice. Further, the flexible distal tip 17 is inserted into the duct so as to follow the intraductal geometry but not cause damage to the duct. As shown in Figure 23C, the distal end 14 has entered the duct. The distal tip 17 continues to follow the intraductal geometry to guide the catheter 12 therethrough. Further, the catheter 12 is more rigid than the distal tip 17 so as to straighten the duct for infusion and collection. Although catheter 12 is described with reference to Figures 23A-23C, other catheters 112, 212, 312, 412, and 512 may be used in a similar fashion.

Figures 24A-24C are schematic diagrams illustrating a method of accessing a breast duct with a catheter body 212 of Figure 21. Referring to Figure 24, the catheter body 212 is prepared to be introduced into a ductal orifice. The practitioner may choose an introducer 218 of a desired flexibility or rigidity. For example, the introducer 218 of different rigidity properties may be inserted for adjusting to a desired tip characteristic during insertion of the distal tip 217 into the breast duct. This arrangement creates a composite insertion member for accessing the breast duct. The desired tip characteristic may relate to the stiffness and flexibility, which may be functions of a material property or a cross-sectional shape. One of skill in the art would recognize that the composite insertion member may have various shapes and material properties to vary the section modulus.

In particular, the tip 217 may be selectively made rigid to penetrate the duct orifice. In this configuration, the length of the catheter 212 and distal tip 217 is relatively rigid. In Figure 24B, the introducer 218, which is rigid or somewhat rigid, has been removed from the distal tip 217 while the catheter 212 is inserted in the duct. In this removed configuration, the catheter 212 may flex and bend through the intraductal geometry. If desired, an introducer 218, which is less stiff, may be inserted into the distal tip 217. With or without an introducer 218, the catheter 212 is advanced further into the duct. In Figure 24C, once a desired position is reached within the duct, a rigid or somewhat rigid introducer 218 may be inserted and advanced into the catheter 212 to straighten the duct for infusion and collection. In a shape memory embodiment, the stiffness or flexibility of introducer 218 may be adjusted in response to the addition of stimulus or removal of a stimulus without removal from the catheter 212. While not shown, it should be recognized that distal tip 217 could be inserted into the ductal orifice without the need of an introducer 218. The introducer of an appropriate stiffness may be inserted into the distal tip 217 during or after intraductal entry of the catheter 212. Although catheter 212 is described with reference to Figure 24A-24C, other catheters 112, 312, 412 and 512 may be used in a similar fashion.

As illustrated in Figures 19 and 20, the catheter 112 may be tapered along its length to make a smooth transition with a received introducer 118 so that a perceptible transition between the catheter 112 and the introducer 118 that would cause any pain to the patient is not formed and felt by the patient. The catheter 112 may also include an atraumatic distal tip portion 114 at its distal end. The distal tip portion 114 may be tapered, contoured and/or rounded so as to produce an atraumatic tip that will reduce or eliminate trauma to the duct upon entry through the ductal orifice and introduction into the ductal lumen past the ductal sphincter. The distal tip portion 114 may also reduce or eliminate trauma upon withdrawal of the catheter 112 from the duct after the medical procedure, such as ductal lavage or the infusion of a diagnostic and/or treatment agent, has been completed. The tip portion 114 may be composed of a soft polymeric material, e.g. including polyvinyl chloride, polyethers, polyamides, polyethylenes, polyurethanes, copolymers thereof and the like. The tip portion 114 may have a diameter in the range from about 0.012 inches (about 0.31 mm) to about 0.020 inches (about 0.51 mm). In an embodiment, the tip portion 114 has a diameter in the range from about 0.014 inches (about 0.36 mm) to about 0.018 inches (about 0.46 mm). The length of the tip portion 114 (extending from the distal end of the distal portion of the catheter toward the proximal end of the catheter) may be in a range from about 0.10 inch (about 0.25 cm) to about 1.0 inch (about 2.5 cm), more typically in the range from about 0.20 inch (about 0.50 cm) to about 0.70 inch (about 1.8 cm).

Figures 28A, 28B, and 28C depict three different formats of the distal end of an introducer 618. The devices shown in Figures 28A to C do not form part of the invention. Referring to Figure 28A, the device is shown in a catheter 612 comprising an elongated internal lumen extending substantially parallel to a longitudinal axis of the catheter and adapted to slideably receive a ductal introducer 618. The distal tip portion 614 of the catheter 612 may be contoured and/or rounded so as to produce an atraumatic tip to form an opening through which the ductal introducer 618 may pass. Such a contour will reduce or eliminate trauma to the duct upon entry through the ductal orifice and introduction into the ductal lumen past the ductal sphincter. The distal tip portion 614 may also reduce or eliminate trauma upon withdrawal of the catheter 612 from the duct after the medical procedure, such as ductal lavage or the infusion of a diagnostic and/or treatment agent, has been completed. The tip portion 614 may be composed of a soft polymeric material, e.g. including polyvinyl chloride, polyethers, polyamides, polyethylenes, polyurethanes, copolymers thereof and the like. The ductal introducer 618 which passes through the internal lumen of the catheter 612, may extend past the distal tip of the catheter 612 and is of sufficient length such that it may penetrate through the ductal orifice and into the ductal lumen past the ductal sphincter prior to the distal tip of the catheter 614 passes through the ductal orifice. The extended length of the probe introducer 618 provides ease of use as well as increasing patient comfort during the ductal lavage procedure. The extended probe introducer 618 may be used to identify the ductal path as well as to introduce the catheter 612 into the ductal orifice. Previously, the ductal lavage process used a separate device (dilator) to identify the ductal path prior to the insertion of the catheter. The extra device and extra procedural step resulted in additional cost, time, as well as an increase in patient anxiety or discomfort due to an additional insertion step to relocate the ductal orifice and ductal path. The increased length of the probe introducer allows the practitioner to locate the ducal path prior to catheter insertion. The catheter may then be inserted into the ductal path by slipping it over the probe introducer without withdrawing the introducer. In an embodiment of the present invention, the length of the ductal introducer that extends beyond the distal tip of the catheter shown in Figure 28A is at least 0.64 mm (0.025 inches).

In Figure 28B, the introducer 618 extends beyond the distal tip of the catheter 614 at least 1.25 mm (0.0492 inches).

In Figure 28C, the introducer 618 extends beyond the distal tip of the catheter 614 at least 2.50 mm (0.0984 inches).

Figures. 29A, 29B, and 29C depict three different formats of the distal end of a catheter 712. The devices shown in Figures 29A to C do not form part of the present invention. Referring to Figure 29A, the device is shown in a catheter 712 comprising an elongated internal lumen extending substantially parallel to a longitudinal axis of the catheter and adapted to slideably receive a ductal introducer 718. The distal tip portion 714 of the catheter 712 may be contoured and/or rounded so as to produce an atraumatic tip to form an opening through which the ductal introducer 718 may pass. Such a contour will reduce or eliminate trauma to the duct upon entry through the ductal orifice and introduction into the ductal lumen past the ductal sphincter. The present invention also provides a step transition from the distal tip portion 714 of the catheter to a probe introducer sheath 701. The distal tip portion of the sheath 701 may be contoured and/or rounded so as to produce an atraumatic tip to form an opening through which the ductal introducer 718 may pass. Such a contour will reduce or eliminate trauma to the duct upon entry through the ductal orifice and introduction into the ductal lumen past the ductal sphincter. The distal tip portion may also reduce or eliminate trauma upon withdrawal of the catheter 712 from the duct after the medical procedure, such as ductal lavage or the infusion of a diagnostic and/or treatment agent, has been completed. The tip portion may be composed of a soft polymeric material, e.g. including polyvinyl chloride, polyethers, polyamides, polyethylenes, polyurethanes, copolymers thereof and the like. The ductal introducer 718 that passes through the internal lumen of the catheter 712 and the sheath 701, may extend past the distal tip of the sheath and is of sufficient length such that it may penetrate through the ductal orifice and into the ductal lumen past the ductal sphincter prior to the distal tip of the sheath passes through the ductal orifice. The length of the ductal introducer that expends beyond the distal tip of the sheath shown in Figure 29A is at least 6.5 millimeters.

In Figure 29B, the introducer 718 extends beyond the distal tip of the sheath 701 at least 12.5 millimeters.

In Figure 29C, the introducer 718 extends beyond the distal tip of the sheath 701 at least 25.5 millimeters.

Figures 30A, 30B, and 30C depict three different formats of the distal end of a catheter 812. The devices shown in Figures 30A to C do not form part of the present invention. Referring to Figure 30A, the device is shown in a catheter 812 comprising an elongated internal lumen extending substantially parallel to a longitudinal axis of the catheter and adapted to slideably receive a ductal introducer 818. The distal tip portion 814 of the catheter 812 may be contoured and/or rounded so as to produce an atraumatic tip to form an opening through which the ductal introducer 818 may pass. Such a contour will reduce or eliminate trauma to the duct upon entry through the ductal orifice and introduction into the ductal lumen past the ductal sphincter. The present invention also provides a step transition from the distal tip portion 814 of the catheter to a probe introducer sheath 801. The distal tip portion of the sheath 801 may be contoured and/or rounded so as to produce an atraumatic tip to form an opening through which the ductal introducer 818 may pass. Such a contour will reduce or eliminate trauma to the duct upon entry through the ductal orifice and introduction into the ductal lumen past the ductal sphincter. The distal tip portion may also reduce or eliminate trauma upon withdrawal of the catheter 812 from the duct after the medical procedure, such as ductal lavage or the infusion of a diagnostic and/or treatment agent, has been completed. The tip portion may be composed of a soft polymeric material, e.g. including polyvinyl chloride, polyethers, polyamides, polyethylenes, polyurethanes, copolymers thereof and the like. The probe introducer 801 sheath may extend beyond the distal end of the catheter 812. The extended probe introducer sheath facilitates a smooth transition and entry of the catheter 812 into the ductal orifice. The length of the probe introducer sheath 801 that extends beyond the distal tip of the catheter 812 shown in FIG 16A is at least 1.5 millimeters.

In Figure 30B, the probe introducer sheath 801 extends beyond the distal tip of the catheter 812 at least 6.0 millimeters.

In Figure 30C, the probe introducer sheath 801 extends beyond the distal tip of the catheter 812 at least 18.5 millimeters.

The probe introducer sheath 801 and the introducer 818, may be combined into a single unit (not shown). The combined sheath/introducer may be formed of a stiff material such as a metal wire or a flexible material such as plastic. The combined sheath/introducer may be formed of Nitinol due to its flexibility, durability, and biocompatibility. The combined sheath/introducer may be constructed from a Nitinol wire that is ground to include a tapered small diameter tip. In an alternative embodiment, the combined sheath/introducer may be formed of multiple materials or the same materials having different stiffnesses. As a result, the combined sheath/introducer may have sections that are more flexible than adjacent sections of the same combined sheath/introducer. As a result, for example, the combined sheath/introducer may have a first, stiff portion for guiding the combined sheath/introducer within the ductal lumen and a second, flexible portion that allows the combined sheath/introducer to conform to the shape of the ductal lumen or lumen branch into which it is introduced. In any of the above-discussed embodiments, the combined sheath/introducer may be coated with a liquid or dry lubricant material that reduces the friction between the combined sheath/introducer and the breast duct during the introduction and advancement of the combined sheath/introducer in the duct.

The combined sheath/introducer may be made of metal or plastics, including shape memory metals and plastics, and may have a tapered and/or an atraumatic tip for gently probing and accessing a breast duct. Preferably, a tapered tip will extend distally of the catheter during the introduction of the catheter into the breast duct After access of the duct is complete, the catheter may be slipped over the combined sheath/introducer and positioned at a location distal to the ductal sphincter.

The catheters as described herein may be constructed of a wide range of appropriate materials for medical use. In a preferred construction, the catheter is formed with PTFE and coated with STS Slipcoat to reduce friction on the device during placement in the breast duct. In lieu of PTFE, alternate materials for the catheter may include but are not limited to: polycarbonate; stainless steel (300 Series); polymide; FEP; PEEK; polyethylene; and PEBAX. Specific dimensional values of catheters are shown in Figure 26, but are provided by way of example. Nevertheless, other appropriate materials and dimensional values may be employed.

The hub 22 housing may be molded from a polycarbonate. The tubing set 25 may be made of PVC with luer connectors made of polycarbonate. The tubing set may be made of a variety of conventional materials including but not limited to: silicone; low-density polyethylene; PVC; tygon; or polypropylene.

In one manufacturing process, the catheters according to one or more aspects of the present invention may be constructed by using an extrusion process. After the process, the distal end of catheter is shaped by molding and/or cutting. An optional, bevel may be is cut to provide the transition between the distal tip portion and the catheter. The edges of the catheter may be rounded using an RF tipping process known to one of ordinary skill in the art. The catheter may be bonded to the hub using a desired medical grade UV adhesive. In turn, the tubes may be bonded to the hub and to the luer connector using UV adhesive as well. If desired, a pinch clamp may be placed on the outflow tube.

The device of the present invention may be used in a method for obtaining cellular material from a human breast milk duct includes introducing a ductal access device such as devices 12,112,212, 312,412, and 512 having at least one lumen into a duct. A wash fluid may be introduced through the access device internal lumen into the milk duct. In the method, a volume of at least 2 ml of wash fluid may be present within the duct for a preselected time, and then at least a portion of the wash fluid is collected from the duct through the lumen of the access device. The method may further comprise massaging and squeezing the breast tissue after introducing the wash fluid but prior to and/or during collecting a portion, of the wash fluid. Introducing the ductal access device typically comprises positioning a distal end of the catheter distal to the ductal sphincter in the breast duct The access device may include only a single lumen that extends into the duct. The preselected time may be less than one second, but will usually be in the range from one second to one hour.

### A METHOD OF OPENING A DUCTAL SPHINCTER USING CONTROLLED FLUID PRESSURE

In accordance with one aspect of the present invention, a medical device for introducing a fluid into a breast duct is provided. The medical device comprises a catheter that is sized and configured to extend within a breast duct The catheter includes an internal lumen that extends substantially parallel to a longitudinal axis of the catheter. The medical device also comprises a manifold hub connected to and in fluid communication with the catheter. The manifold hub includes at least four ports in fluid communication within an interior of the manifold hub. At least two of the at least four ports are in fluid communication with channels formed within the manifold hub for receiving a fluid introduction line and a ductal fluid collection line. The manifold hub has a height that extends parallel the longitudinal axis of the catheter and a length that extends perpendicular to the longitudinal axis of the catheter. The length of the manifold hub is greater than the height of the manifold hub.

The device of the present invention may comprise a catheter having an outer diameter of about 0.01 inch (0.254 mm) to about 0.05 inch (or 1.27 mm). The catheter may have an inner lumen diameter in the range from about 0.007 inch (or 0.178 mm) to about 0.047 inch (or 1.19 mm). The associated manifold hub may further comprise an infusion connector providing a fluid flow path into the lumen of the catheter from an infusion device; and a collection connector providing a fluid outlet path from the lumen of the catheter to a fluid collection device.

A watertight sealing system may be located at a proximal end of the manifold hub. This sealing system may seal around a dilator or other introducer positioned within and extending through the medical instrument. Such a sealing system may include a Touhy-Borst fitting. A dilator or other introducer member(s) for use with the catheter may have an outer diameter of 0.024 inch (or 0.61 mm) to about 0.001 inch. The introducer may also be tapered.

### BRIEF DESCRIPTION OF THE FIGURES

Figure 1 is a perspective view of a medical instrument according to aspects of the present invention;

Figures 2A and 2B are cross sections of alternative embodiments of the medical instrument illustrated in Figure 1 that do not form part of the present invention;

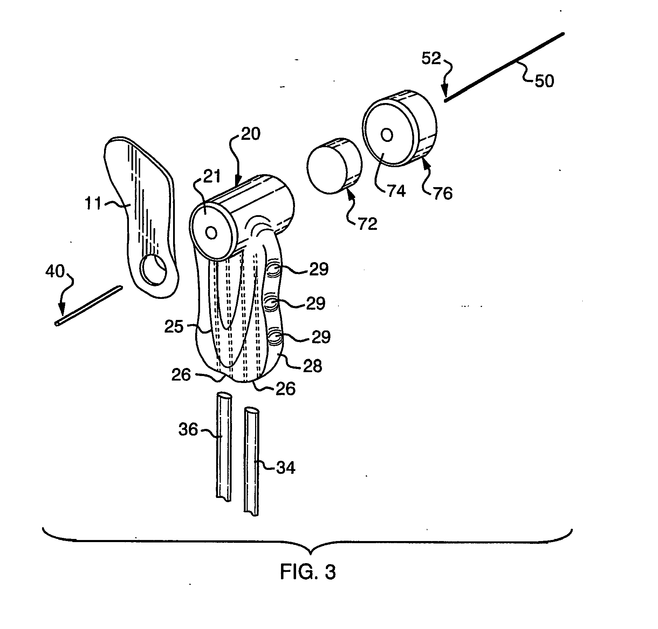

Figure 3 is an explode perspective view of the medical instrument of Figure 1;

Figure 4 is a top view of the medical instrument of Figure 1 with infusion and collection lines extending between a manifold hub and respective infusion and collection devices;

Figure 5 is a side view of the medical instrument illustrated in Figure 1 carrying infusion and collection lines;

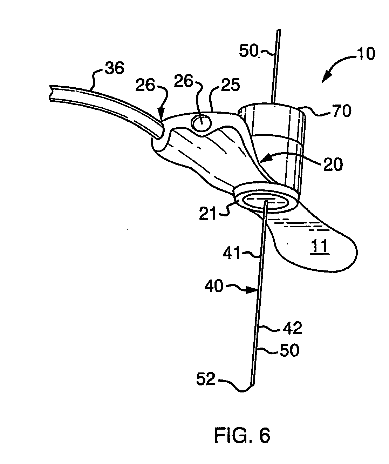

Figure 6 is another perspective view of the medical instrument illustrated in Figure 1;

Figures 7 and 8 illustrate an alternative embodiment of the medical instrument according to aspects of the present invention;

Figures 9-10 illustrate a method for introducing the medical instrument of Figure 1 into a breast duct using a stiff introducer; and

Figures 12-14 illustrate an alternative method for introducing the medical instrument of Figure 1 into a breast duct using a flexible introducer.

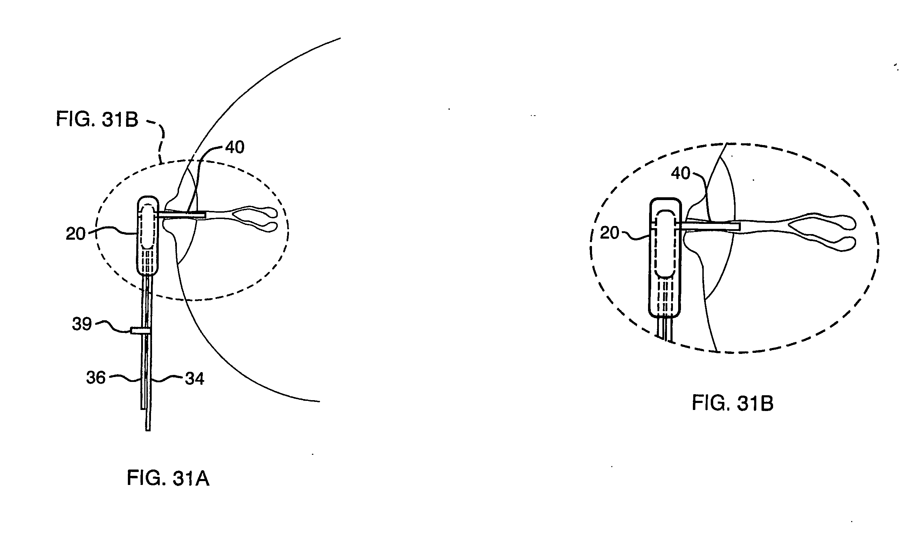

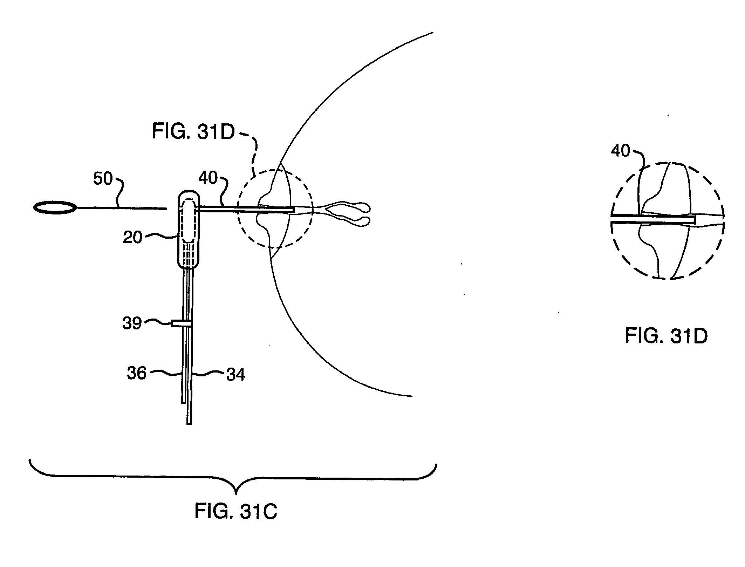

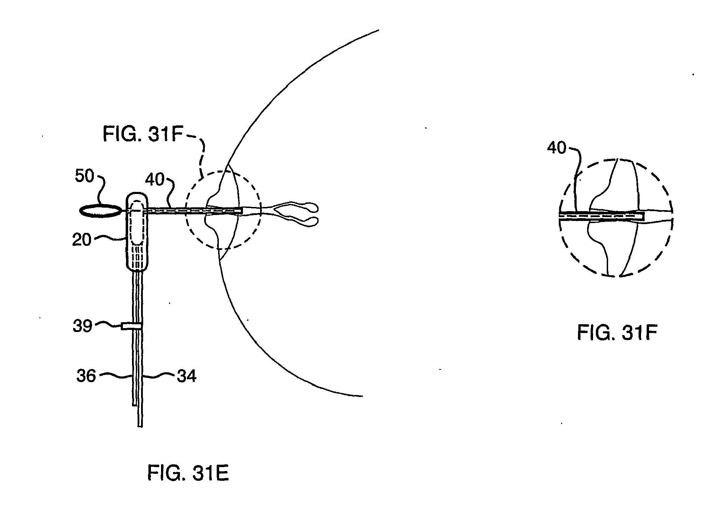

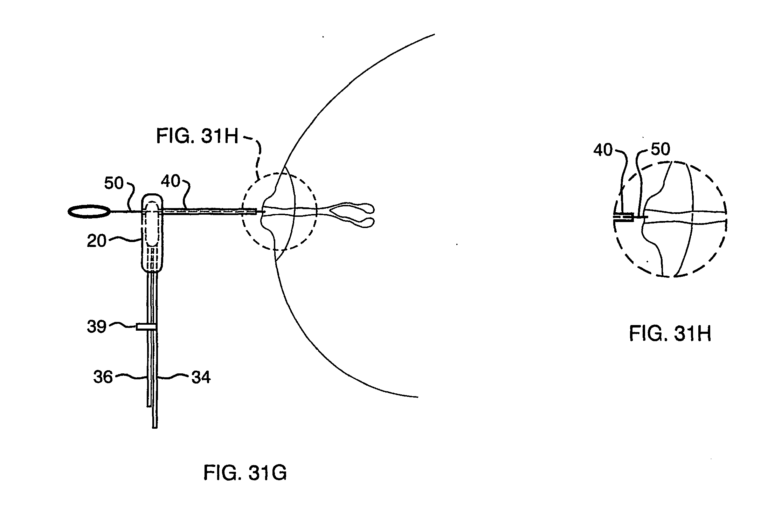

Figures 31A-D illustrates an alternative method of breast microcatheter insertion aided by fluid pressure application.

### DETAILED DESCRIPTION

Figure 1 illustrates an embodiment of a low profile, single lumen medical instrument 10 for performing a medical procedure within a breast duct. As used herein, the phrase "medical procedure" may include preparatory procedures, diagnostic procedures or therapeutic procedures. These procedures could include the steps of delivering material(s) into the breast duct and/or retrieving material(s) from within the breast duct.

The medical instrument 10 may be used to infuse ductal wash fluid delivered to the manifold hub 20 into the breast duct, and collect or draw up ductal fluid samples, including hundreds of ductal epithelial cells and/or cell clusters of greater than ten cells, from within the breast duct for analysis. Furthermore, the medical instrument 10 may be used to infuse a diagnostic agent or therapeutic agent into a breast duct. As shown in Figures 1-8, the instrument 10 may also include a member 11 for securing the instrument 10 to a patient. The securing member 11 may have a biocompatible adhesive on one side for contacting and attaching the instrument 10 to the patient. The member 11 is sized to prevent movement of the manifold hub 20 relative to the body of the patient. As illustrated in Figures 7 and 8, sections 11A and 11B of the member 11 may be folded onto each other so that the size of the securing member 11 may be adjusted to the patient and the forces created during the procedure. Additionally, the member 11 may be positioned distal a spacer 90 (discussed below).

As illustrated in Figures 1-6, the medical instrument 10 includes a manifold hub 20 and a ductal access catheter 40 that extends from a distal end 21 of the manifold hub 20. The access catheter 40 is sized to accesses the breast duct As illustrated, the manifold hub 20 may have a low profile (height) in a direction that extends parallel to the length of the catheter 40. As illustrated in Figures 1 and 3 to 6, the height of the manifold hub 20 is less than its length (the direction it extends perpendicular to the length of the catheter 40). In a first embodiment, the manifold hub 20 may have a width in a range from about 6.35 mm (0.25 inch) to about 9.53 mm (0.375 inch) and a height in a range from about 1.9 cm (0.75 inch) to about 2.54 mm (1.0 inch). In another construction, the manifold hub 20 has an internal fluid capacity of 1 ml or less. The low profile of the manifold hub 20 will help to prevent a pivot point from being formed at a location along the length of the catheter 40 at which a large torque may be applied to the duct of a patient during a medical procedure. By eliminating or, at least, significantly reducing any torquing of the duct, the duct will not be kinked, closed due to a change in the position of ductal tissue or injured due to the catheter 40 pushing against the epithelial lining of the duct. The instrument 10 also has an ergonomic design that allows easily handling and grasping by an attendant or practitioner so that the manifold hub 20 and catheter 40 may be easily manipulated.

In the event that the manifold hub 20 needs to be spaced from the nipple surface, a retractable spacer 90 may be positioned on the distal end of the manifold hub and at the proximal end of the catheter 40 as shown in Figures 7 and 8. The retractable spacer 90 may have a spacing distance in the range from about 1mm to about 10mm, most typically in the range of about 5mm. In a first embodiment, the retractable spacer 90 may include a first spacing member 92 received within a second spacing member 93. Alternatively, the first spacing member 92 may telescopically receive the second spacing member 93. In this construction, the first member 92 is secured against movement relative to the manifold hub 20, while the second member 93 is moveable relative to both the first member 92 and the manifold hub 20. Alternatively, both of the spacing members 92, 93 are moveable relative to the manifold hub 20. The distances required for spacing the manifold hub 20 from the surface of the nipple, for example distances of between about 15 mm and 20 mm, may be controlled by locking the spacing members 92, 93 in an extended position (Figure 8). Alternatively, the retractable spacer 90 may be locked in a retracted position (Figure 7). Alternatively, the retractable spacer 90 includes a single moveable spacing member 95 that slideably receives a portion of the manifold hub 20 to achieve the retracted position and that may be locked at the end of the manifold hub 20 to achieve the extended position. In any of the above-discussed embodiments, the spacing members 92, 93, 95 may be rotated relative to each other and the manifold hub 20 in order to lock each spacing member 92, 93 and 95 against translational movement relative to each other and the manifold hub 20. Any known rotational locking system for telescoping members may be used. Alternatively, the spacing members 92, 93, 95 may be snapped into a locked position using well known snap locks. When additional spacing is needed, members 92, 93, 95 of different sizes or more than two telescoping members may be provided.

The manifold hub 20 may be formed of a transparent material so that an attendant or practitioner may easily view fluid(s) and material(s) within the manifold hub 20. The transparent material may be a plastic, such as ABS plastics or other known plastic materials. As illustrated in Figures 1-8, an embodiment of the manifold hub 20 may have a substantially "F" shape.

As shown in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. The connected infusion device 38 may include a syringe or other known fluid containers. The infusion device 38 may include a fluid receptacle, such as a bag or a container, positioned at a location above the breast of the patient. The height of the container above the breast of the patient and gravity are used to deliver the fluid from the infusion device 38 to the infusion tube 34. The embodiments shown in Figure 2A and 2B do not form part of the invention

As shown in Figures 2A, 2B and 4, the manifold hub 20 also includes a second port 32 for connection to a collection tube 36. Ductal fluid samples collected from within the breast duct may be delivered from the manifold hub 20 to a collection receptacle via the collection tube 36. The collection receptacle may include a syringe or other known fluid collection device including a medical fluid bag or container. The collection receptacle may include or be connected to a source of negative pressure so that an area of low pressure may be created within the collection tube 36 and, if needed, the manifold hub 20 for assisting in the delivery of the retrieved ductal fluid sample to the collection receptacle. The area of low pressure, for example a vacuum, may be created using a bulb syringe, a hand-operated vacuum source, a foot operated vacuum source or motor controlled vacuum source. These vacuum sources may include a pump that creates negative pressure within the collection tube 36. In addition to a source of lower pressure, or in place of the source of lower pressure, low pressure within collection tube 36 may be created by infusing fluid into the manifold hub 20, thereby increasing the pressure within the manifold hub 20 relative to the collection tube 36. In any of these constructions, the collection receptacle may be positioned at a location below the patient during the procedure so that the collected ductal fluid sample may be delivered to the receptacle by gravity.

The first and second ports 30, 32 may include an opening along the sidewall of the manifold hub 20 that is round, oval or any other geometric shape conductive to fluid flow either into the duct or out from the duct as shown in Figures 2A and 2B. The diameter of the ports 30, 32 may be that diameter which is suitable to achieve a desired flow rate into the duct or aspiration or collection rate out from the duct. Thus, the diameters of the ports 30, 32 may be in a range from about 0.060 inches to about 0.090 inches. One side port 30, 32 may be larger or smaller than the other, especially where such differential port size provides a desired flow rate into or out from one of the lumens, or an overall lavage efficiency of infusion and aspiration or collection of lavage and ductal fluid. The embodiments shown in Figure 2A and 2B do not from part of the invention.

Figures 1-6 illustrate that the first and second ports 30, 32 and their associated tubes 34, 36, respectively, are positioned within a connector housing 25 that extends transverse to the longitudinal axis of the manifold hub 20 and the catheter 40. The connector housing 25 may be integrally formed with the manifold hub 20 as a unitary element Alternatively, the connector housing 25 may be formed separate of the manifold hub 20 and secured to the manifold hub 20 as discussed below with respect to the catheter 40. The connector housing 25 includes two channels 26 that each receives one of the tubes 34, 36. The channels 26 extend from the ports 30, 32 on the manifold hub 20 to the outer, end surface of the connector housing 25. Each channel 26 aligns the received tube 34, 3 6 with its respective manifold hub port 30, 32 for easy, reliable and quick connection of the tubes 34, 36 to their respective ports 30, 32. The channels 26 also support the tubes 34, 36 at their point of connection to their ports 30, 32 so that the tubes 34, 36 do not create a moment (that may torque the duct) about the point where they connect to their respective ports 30, 32. The connector housing 25 may also include contoured sidewalls 28 with integrally formed or otherwise secured ridges 29 that may be gripped by an attendant or a practitioner. The contoured sidewalls 28 permit easy grasping by the attendant or practitioner and allow the attendant or practitioner to orient the instrument 10 during a procedure without looking at the instrument 10.

As shown in Figure 2B, the first and second ports 30, 32 may include posts 35 that extend within connector housing 25 and receive the flexible infusion and collection tubes 34, 36. The infusion and collection tubes 34, 36 may be formed of flexible tubing such as surgical tubing. However, the tubes 34, 36 may be formed of any flexible material including a flexible plastic. The tubes 34, 36 are formed of flexible PVC. The embodiments shown in Figure 2A and 2B do not form part of the invention.

The posts 35 may securely receive the tubes 34, 36 when the tubes 34, 36 are positioned over, or within, the posts 35. Alternatively, the ports 30, 32 and their associated posts 35 may include luer lock fittings (not shown) that cooperate with corresponding luer lock fittings on a first end of the tubes 34, 36. The second end of the tubes 34, 36 may also include luer lock fittings that mate with standard luer lock fittings on the syringes or other fluid containers. The luer lock fittings may be either male or female fittings. As discussed herein, the syringes and other fluid containers may carry and infuse saline, diagnostic materials, such as contrast materials, and therapeutic treatment materials into the infusion tube 34. The tubes 34, 36 may be secured to the posts 35 of the manifold hub 20 and the luer locks using a UV curable adhesive or other known bonding agents. Alternatively, the tubes 34, 36 may be secured to the manifold hub 20 and the luer locks by overmolding.

As shown in Figures 1-6, the first port 30 may be positioned adjacent the second port 32 along the perimeter of the manifold hub 20. In the illustrated embodiment, the circumferentially adjacent ports 30, 32 are spaced the same longitudinal distance from the first and second ends 22, 24 of the manifold hub 20. This spacing of the ports 30, 32 provides for a compact and low profile manifold hub 20 that, as discussed above, will not create a moment and associated forces that torque the duct when the manifold hub 20 is positioned on the patient and free of support from a practitioner or other attendant.

The second port 32 may be circumferentially spaced any distance from the first port 30 around the wall of the manifold hub 20. The first port 30 may be between forty-five and ninety degrees offset from the second port 32 around the circumference of the manifold hub 20. Alternatively, the first port 30 may be circumferentially offset along the manifold wall from the second port 32 by between ninety and one hundred-eighty degrees. The first port 30 is circumferentially offset from the second port 32 by about one hundred-eighty degrees so that the first and second ports 30, 32 oppose each other within the manifold hub 20.

Alternatively, it is possible for the second port 32 to be located along the hub 20 at a position that is spaced a greater longitudinal distance away from the catheter 40 than the first port 30. The second port 32 may be used to collect the fluid that enters the manifold hub 20 from the collection catheter 40. For example one port may be located about 2.0 cm from the distal tip of the catheter 40 and one port may be located about 2.5 cm from the distal tip of the catheter 40.

The tubes 34 and 36 may each include a one-way check valve 39 (Figure 2A) to control the fluid flow into and out of the manifold hub 20. The check valve 39 in the tube 34 may prevent, for example, wash fluid from flowing back into a syringe connected to tube 34 after being infused into tube 34. Similarly, check valve 39 in tube 36 may be used to prevent retrieved ductal fluid samples in tube 36 from flowing back into the manifold hub 20. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used. The embodiments shown in Figure 2A and 2B do not form part of the invention.

As shown in Figures 1-6, the catheter 40 includes a thin walled microcatheter 41 that is secured to the manifold hub 20. In a first construction, the microcatheter 41 is integrally formed as part of the manifold hub 20. In another construction, the microcatheter 41 is formed as a separate piece and then secured to the manifold hub 20 by microwelding or a UV curable adhesive. Other known techniques for securing the microcatheter 41 to the manifold hub 20 could be used. Moreover, the catheter 40 may be coated with a known agent to provide a lubricious coating that allows it to be easily introduced into the breast duct openings. The coating may include a lubricant, a cleaning agent, anesthetic and/or a dilating agent. The microcatheter 41 may be formed of any known biocompatible material such as FEP. The catheter 40 may have an outer diameter in a range from about 0.01inch (about 0.25 mm) to about 0.05 inch (about 1.25 mm) with an inner lumen 43 having a diameter in the range from about 0.008 inch (about 0.2 mm) to about 0.047 inch (about 1.2 mm). Furthermore, the microcatheter 41 may have an inner lumen 43 having an outer diameter of about 0.030 inch (about 0.762 mm) and an inner diameter of about 0.025 inch (about 0.63 mm).

The catheter 40 may include length indicia (not shown) on an outer surface of the catheter 40 that permits a user to determine the depth to which the distal end of the catheter has been introduced into the breast duct. Alternatively, the catheter 40 could include an integrally formed or attached stop element (not shown) that prevents insertion of the catheter into the duct beyond a predetermined distance. The stop element may comprise a knob on the catheter 40 having an increased diameter for preventing the distal portion of the catheter 40 from entering a duct a greater distance than the knob is spaced from the distal end of the catheter40.

As illustrated in Figures 1-7, the catheter 40 may be tapered along its length to make a smooth transition with a received introducer 50 so that a perceptible transition between the catheter 40 and the introducer 50 that would cause any pain to the patient is not formed and felt by the patient. The catheter 40 may also include an atraumatic distal tip portion 42 at its distal end. The distal tip portion 42 may be tapered, contoured and/or rounded so as to produce an atraumatic tip that will reduce or eliminate trauma to the duct upon entry through the ductal orifice and introduction into the ductal lumen past the ductal sphincter. The distal tip portion 42 may also reduce or eliminate trauma upon withdrawal of the catheter 40 from the duct after the medical procedure, such as ductal lavage or the infusion of a diagnostic and/or treatment agent, has been completed. The tip portion 42 may be composed of a soft polymeric material, e.g. including polyvinyl chloride, polyethers, polyamides, polyethylenes, polyurethanes, copolymers thereof and the like. The tip portion 42 may have a diameter in the range from about 0.012 inches (about 0.31 mm) to about 0.020 inches (about 0.51 mm). In a construction, the tip portion 42 has a diameter in the range from about 0.014 inches (about 0.36 mm) to about 0.018 inches (about 0.46 mm). The length of the tip portion 42 (expending from the distal end of the distal portion of the catheter 40 toward the proximal end of the catheter 40) may be in a range from about 0.10 inch (about 0.25 cm) to about 1.0 inch (about 2.5 cm), more typically in the range from about 0.20 inch (about 0.50 cm) to about 0.70 inch (about 1.8 cm).

The stiffened distal portion of the catheter 40, including the distal tip 42, may have an average bending stiffness in the range from about 0.001 cm-kg (0.010 inch-lbs) to about 0.576 cm-kg (0.5 inch-lbs). The catheter 40 may also have a stiffness that is similar to that of a heavy suture (approximately 0.025 OD). The proximal portion of the catheter 40 may have a cross-sectional geometry and/or size that inhibits insertion through the ductal orifice and into the ductal lumen.

A Touhy-Borst fitting 70 may be positioned at a proximal end 22 of the manifold hub 20 to allow a user to easily receive and move the catheter 40 over an introducer 50 as shown in Figures 1-6. The Touhy-Borst fitting 70 is positioned at the end of the manifold hub 20 to cover and seal the opening 77 through which an introducer 50 (discussed-below) including a guidewire, stylet, dilator or the like may extend. The Touhy-Borst fitting 70 comprises a silicone plug 72 including a small aperture 74 for receiving the introducer 50, and a threaded cap 76. When the cap 76 is rotated in a first direction, the silicone plug 72 is altered and the size of the aperture 74 is reduced. This results in the silicone plug 72 forming a seal around the inserted introducer 50. When the cap 76 is turned in a second, opposite direction, the aperture 74 and created seal open, thereby allowing the introducer 50 to be removed. The silicone plug 72 may also be closed to seal the proximal end of the manifold hub 20 so when the introducer 50 is not present so that the distal end of the manifold hub 20 is sealed against fluid flow when the proximal end 22 is free of an introducer 50.

The introducer 50 may be located within the manifold hub 20 to assist in placing the catheter 40 into the breast duct and ductal lumen via the ductal opening as shown in Figure 1. The introducer 50 may include a tapered dilator, a series of progressively larger diameter dilators, a guidewire, including tapered guidewires, a stylet or other known introducers. As illustrated, the introducer 50 will pass through the Touhy-Borst fitting 70 at the proximal end 22 of the manifold hub 20 so that the introducer 50 may be removed after positioning of the catheter 40 and prior to the infusion/collection of the wash fluid. As discussed above, prior to being inserted into the breast duct, the Touhy-Borst fitting 70 may be turned down over the introducer 50 during introduction and then backed off when the catheter 40 has been positioned within the breast duct to the desired depth. The introducer 50 may be formed of a stiff material such as a metal wire or a flexible plastic cord. Furthermore, the introducer 50 may be formed of stainless steel or a flexible material such as polypropylene monofilament. Alternatively, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. The introducer 50 may be coated with a liquid or dry lubricant material that reduces the friction between the introducer 50 and the breast duct during the introduction and advancement of the introducer 50 in the duct

The introducer 50 may be made of metal or plastics, including shape memory metals and plastics, and may have a tapered and/or an atraumatic tip for gently probing and accessing a breast duct. Preferably, a tapered tip 52 will extend distally of the catheter 40 during the introduction of the catheter 40 into the breast duct. After access of the duct is complete, the introducer 50 may be withdrawn, the Touhy-Borst fitting 70 may be closed and the indwelling catheter 40 may be positioned at a location distal to the ductal sphincter. The introducer 50 may have an outer diameter of from about 0.13 mm (0.005 inch) to about 0.76 mm (0.030 inch). In a construction, the introducer 50 has an outer diameter of about 0.25 mm (0.010 inch). The introducer 50 may extend through the manifold hub 20 and the lumen of the catheter 40. The introducer 50 may be tapered over its length.

During the process of introducing the catheter 40 into the duct, a ductal opening is located on the surface of a nipple by a practitioner or attendant and a first introducer 50 is advanced through the ductal opening into the duct The introducer 50 may be a long flexible guide wire, a shorter dilator or any of the other above-mentioned introducers. Prior to, or after the introducer 50 is positioned within the duct, the manifold hub 20 and catheter 40 may receive the first or a second introducer 50. As previously discussed, the Touhy-Borst fitting 70 may be locked about the received introducer 50 to form a fluid tight seal at the distal end of the manifold hub 20 so that fluid does not exit the manifold hub 20 around the introducer 50 during the insertion catheter 40 into the duct (See Figures 9, 10, 12 and 13).

When the catheter 40 is positioned within the duct as intended, the Touhy-Borst fitting 70 is opened and the introducer 50 removed (See Figure 14). The Touhy-Borst 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

A method of lavage may include seating a patient substantially upright in a chair during the lavage procedure, rather than the standard or classic supine (face up) position. Alternatively, the patient may be lavaged in a prone position, face down, with nipples and breast down. The prone face down position takes advantage of gravity and allows the breast ducts to drain into the collection receptacle during the procedure when the outflow port is open. Thus, as discussed above, the lavaging procedure may include infusing the breast duct with a wash fluid through an open inflow lumen while an outflow lumen is closed; closing the inflow lumen when the duct is filled; squeezing or massaging the breast or both; and opening the outflow lumen to collect the wash fluid.

The cells collected may comprise ductal epithelial cells; the ductal fluid collected may comprise molecular and cellular material. Analysis of the ductal epithelial cells and/or the molecular and cellular material in the ductal fluid may proceed as described below discussing the diagnostic potential of these collected materials. The collected cells and fluid and fluid components may be analyzed. The lavage fluid including the ductal cells may be analyzed for diagnostic purposes. Conditions in a breast milk duct that are desirable to diagnose include a cancer or precancer condition. The precancer condition may include atypical ductal hyperplasia (ADH) or low-grade ductal carcinoma in situ (LG-DCIS). The diagnostic agent may also have the ability to diagnose other breast related conditions, including, e.g. fibrotic, cystic or conditions relating to lactation. Diagnostic agents may be mixed with the ductal fluid (either in the lavage procedure, or after the fluid is collected).

The fluid infused into the duct to lavage the duct may include known, biocompatible fluids. These lavage fluids may include saline, phosphate buffered saline, a nonabsorbable fluid, an isotonic solution, an osmotic solution, a hypotonic solution, and a hypertonic solution. The wash fluid may comprise for example, saline, phosphate buffered saline, a nonabsorbable fluid, an isotonic solution, an osmotic solution, a hypotonic solution, a hypertonic solution.a protein, a colloid, a sugar, a polymer, mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, a synthetic colloid, an antibody, a binding protein, or albumin.

As mentioned above, a diagnostic or therapeutic agent may be introduced into a breast duct through the manifold hub 20 and catheter 40. The introduced agent for infusing into the duct may comprise a non-absorbable fluid and/or an oncotic agent and/or an osmotic agent. The agent may be soluble. The agent may comprise a molecule that is a protein, a colloid, a sugar, or a polymer. The agent may be mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, or a synthetic colloid. The agent may comprise a protein and the protein may be a binding protein or an antibody. The binding protein may be albumin. Administering may comprise administering locally, and local administration may comprise administering intraductally. A system for increasing or standardizing an amount of fluid collectable from a milk duct of a breast may comprise infusing a nonabsorbable fluid and/or an osmotic agent and/or an oncotic agent into the ductal lumen, a medical tool for delivering the agent to the ductal lumen, and instructions for use.

As shown in Figures 31A-D, the insertion of a medical instrument, such as a catheter, into a breast duct may be aided by fluid pressure. As mentioned above, during the process of introducing the catheter 40 into the duct, a ductal opening is located on the surface of a nipple and an introducer 50 is advanced through the ductal opening into the duct (See Figures 9, 10, 12 and 13). Once the introducer 50 has located a duct, the catheter 40 is introduced into the ductal orifice just distal to the ductal sphincter. At this point, the introducer 50 may be removed and fluid pressure applied through the catheter. The use of fluid pressure would aid in opening the ductal sphincter as well as opening, straightening, and lubricating during the insertion of the catheter 40 into the duct. Once the catheter 40 has been seated properly within the duct, the fluid pressure is discontinued.

As shown previously in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. The infusion device 38 may also be used to apply fluid pressure through the catheter 40 to assist the penetration of the ductal sphincter. The connected infusion device 38 may include a syringe, a pump, or other known fluid containers. In one construction, the connected infusion device 38 is a precision fluid pump that provides either continuous or pulsed fluid pressure. The advantage of using pulsed fluid pressure would be that fluid pressure could be controlled with an incremental advancement of the catheter, thus preventing a build up of pressure within a breast duct Activation of the pump may be achieved by multiple means such as a toggle switch, a push button, a foot pedal or other methods known to one skilled in the art. In yet another construction, the infusion device 38 may include a fluid receptacle, such as a bag or a container, positioned at a location above the breast of the patient In this construction, the height of the container above the breast of the patient and gravity are used to deliver the fluid from the infusion device 38 to the infusion tube 34. In still another construction, the fluid is warmed to body temperature or above to relax the ductal sphincter and milk duct tissues to assist in inserting the catheter. The embodiments shown in Figure 2A and 2B do not form part of the invention.

As mentioned above, the device of the present invention may include a one-way check valve 39 (Figure 4) to control the fluid flow into and out of the manifold hub 20. The check valve 39 in the tube 34 may prevent, for example, fluid from flowing back into the infusion device 38 when fluid pressure is being applied. Similarly, check valve 39 in tube 36 may be used to prevent fluid in the manifold hub 20 from flowing back into the collection device 39. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used.

### DUCTAL LAVAGE MICROCATHETER WITH USER ACTIVATED VALVE AND NITINOL INTRODUCER

The breast is a specialized, glandular structure including a system of complicated breast ducts that radiate from the nipple and that are bound together by fairly dense connective tissue. Each of these breast ducts includes an associated ductal orifice on the surface of a nipple through which ductal fluid may be expressed. Each duct includes a series of successive interlobular branches that drain through the main, lactiferous branch, which terminates and exits the breast at the nipple via the associated ductal orifice. Immediately proximate the ductal orifice, each lactiferous duct includes a lactiferous sinus in which ductal fluid may accumulate. A ductal sphincter resides within the lactiferous sinus and prevents ductal fluid from unintentionally exiting the breast duct through its associated ductal orifice.

Breast cancer is believed to begin in the lining of these breast ducts. For several decades significant members of the medical community dedicated to studying breast cancer have believed and shown that the cytological analysis of cells retrieved from nipple discharge fluid from within breast ducts may provide valuable information leading to identifying patients at risk for breast cancer. Indeed, Papanicolaou contributed to the genesis of such a possibility of a "Pap" smear for breast cancer by analyzing the cells contained in nipple discharge. More recently, cancer specific markers have been detected in ductal fluid obtained by nipple aspiration. However, the retrieval techniques and instruments used by these members of the medical community did not routinely obtain meaningful ductal fluid samples.

In their attempts to retrieve the breast duct fluid sample including ductal epithelial cells, practitioners introduced wash fluids into a breast duct using indwelling hair-like single lumen catheters. After the fluid was introduced into the duct, the fluid introduction catheters were removed. Then, externally applied nipple aspiration techniques or external pressure applied to the breast were used to collect samples of the ductal fluid. However, these techniques required that significant, sometimes painful, pressure be created on the nipple surface or along the sides of the breast to overcome the fluid retaining properties of the ductal sphincter. Also, these techniques did not routinely provide meaningful ductal fluid samples with a sufficient number of ductal epithelial cells for a meaningful cellular analysis. These techniques typically caused the recovery of samples with twenty or fewer ductal epithelial cells. Additionally, these techniques did not provide samples with cell clusters of 10 or more cells. As a result, the obtained fluid samples could not consistently provide an accurate indication of whether or not the duct from which they were retrieved included precancerous or cancerous cells. Consistent, meaningful ductal epithelial cell samples have been provided by the medical instrument disclosed in U.S. Patent No. 6,413,228 to Hung et al..

Other medical instruments, such as those used during galactography, are introduced into the breast duct in order to visually determine the presence of cancerous cells within a breast duct. However, these devices typically extend a significant distance out of the breast duct during the performed procedure. These distances may be twelve inches or greater. As a result, when an operator is not holding the tool, the moment created by the weight and length of the section of the instrument extending out of the duct may cause the indwelling portion of the instrument to engage the sidewalls of the duct, torque and/or kink the duct and distort the nipple. These effects on the duct and nipple may impede the procedure by twisting or crimping the indwelling portion of the instrument, possibly injuring the patient's duct and causing significant discomfort to the patient. As a result, a patient must either endure the pain and discomfort caused by these long instruments or an attendant must constantly support the instrument above the patient during the medical procedure.

However, in the confined space around an operating table and in the area surrounding a nipple surface, it is not practical to have an attendant constantly holding the end of the instrument that is extending into the breast duct. Therefore, prior to receiving the procedure, a patient must decide to either experience discomfort during the procedure or choose not to have the procedure performed. Prior art instruments are also not ergonomically designed for easy grasping and adjusting by a practitioner or attendant while acting in the area surrounding a nipple.

### BRIEF DESCRIPTION OF THE FIGURES

Figure 1 is a perspective view of a medical instrument according to aspects of the present invention;

Figures 2A and 2B are cross sections of alternative embodiments of the medical instrument illustrated in Figure 1 that do not form part of the present invention;

Figure 3 is an exploded perspective view of the medical instrument of Figure 1;

Figure 4 is a top view of the medical instrument of Figure 1 with infusion and collection lines extending between a manifold hub and respective infusion and collection devices;

Figure 5 is a side view of the medical instrument illustrated in Figure 1 carrying infusion and collection lines;

Figure 6 is another perspective view of the medical instrument illustrated in Figure 1;

Figures 7 and 8 illustrate an alternative embodiment of the medical instrument according to aspects of the present invention;

Figures 9-10 illustrate a method for introducing the medical instrument of Figure 1 into a breast duct using a stiff introducer, and

Figures 12-14 illustrate an alternative method for introducing the medical instrument of Figure 1 into a breast duct using a flexible introducer.

Figures 31E-H illustrate the medical instrument according to aspects of the present invention including a ductal lavage microcatheter with user activated valve and Nitinol introducer with a ring shaped handle.

### DETAILED DESCRIPTION OF THE INVENTION

Figure 1 illustrates an embodiment of a low profile, single lumen medical instrument 10 for performing a medical procedure within a breast duct. As used herein, the phrase "medical procedure" may include preparatory procedures, diagnostic procedures or therapeutic procedures. These procedures could include the steps of delivering material(s) into the breast duct and/or retrieving material(s) from within the breast duct.

The medical instrument 10 may be used to infuse ductal wash fluid delivered to the manifold hub 20 into the breast duct, and collect or draw up ductal fluid samples, including hundreds of ductal epithelial cells and/or cell clusters of greater than ten cells, from within the breast duct for analysis. The medical instrument 10 may be used to infuse a diagnostic agent or therapeutic agent into a breast duct. As shown in Figures 1-8, the instrument 10 may also include a member 11 for securing the instrument 10 to a patient The securing member 11 may have a biocompatible adhesive on one side for contacting and attaching the instrument 10 to the patient The member 11 is sized to prevent movement of the manifold hub 20 relative to the body of the patient. As illustrated in Figures 7 and 8, sections 11A and 11B of the member 11 may be folded onto each other so that the size of the securing member 11 may be adjusted to the patient and the forces created during the procedure. Additionally, the member 11 may be positioned distal a spacer 90 (discussed below).

As illustrated in Figures 1-6, the medical instrument 10 includes a manifold hub 20 and a ductal access catheter 40 that extends from a distal end 21 of the manifold hub 20. The access catheter 40 is sized to accesses the breast duct As illustrated, the manifold hub 20 may have a low profile (height) in a direction that extends parallel to the length of the catheter 40. As illustrated in Figures 1-6, the height of the manifold hub 20 may be less than its length (the direction it extends perpendicular to the length of the catheter 40). In a first embodiment, the manifold hub 20 may have a width in a range from about 6.35 mm (0.25 inch) about 9.53 mm (0.375 inch) and a height in a range from about 1.9 cm (0.75 inch) to about 2.54 mm (1.0 inch). In another embodiment, the manifold hub 20 has an internal fluid capacity of 1 ml or less. The low profile of the manifold hub 20 will help to prevent a pivot point from being formed at a location along the length of the catheter 40 at which a large torque may be applied to the duct of a patient during a medical procedure. By eliminating or, at least, significantly reducing any torquing of the duct, the duct will not be kinked, closed due to a change in the position of ductal tissue or injured due to the catheter 40 pushing against the epithelial lining of the duct The instrument 10 also has an ergonomic design that allows easily handling and grasping by an attendant or practitioner so that the manifold hub 20 and catheter 40 may be easily manipulated.

In the event that the manifold hub 20 needs to be spaced from the nipple surface, a retractable spacer 90 may be positioned on the distal end of the manifold hub and at the proximal end of the catheter 40 as shown in Figures 7 and 8. The retractable spacer 90 may have a spacing distance in the range from about 1mm to about 10mm, most typically in the range of about 5mm. In a first embodiment, the retractable spacer 90 may include a first spacing member 92 received within a second spacing member 93. Alternatively, the first spacing member 92 may telescopically receive the second spacing member 93. In this construction, the first member 92 is secured against movement relative to the manifold hub 20, while the second member 93 is moveable relative to both the first member 92 and the manifold hub 20. In an alternative construction, both of the spacing members 92, 93 are moveable relative to the manifold hub 20. The distances required for spacing the manifold hub 20 from the surface of the nipple, for example distances of between about 15 mm and 20 mm, may be controlled by locking the spacing members 92, 93 in an extended position (Figure 8). Alternatively, the retractable spacer 90 may be locked in a retracted position (Figure 7). In an alternative construction, the retractable spacer 90 includes a single moveable spacing member 95 that slidably receives a portion of the manifold hub 20 to achieve the retracted position and that may be locked at the end of the manifold hub 20 to achieve the extended position. In any of the above-discussed embodiments, the spacing members 92, 93, 95 may be rotated relative to each other and the manifold hub 20 in order to lock each spacing member 92, 93 and 95 against translational movement relative to each other and the manifold hub 20. Any known rotational locking system for telescoping members may be used. Alternatively, the spacing members 92, 93, 95 may be snapped into a locked position using well known snap locks. When additional spacing is needed, members 92, 93, 95 of different sizes or more than two telescoping members may be provided.

The manifold hub 20 may be formed of a transparent material so that an attendant or practitioner may easily view fluid(s) and material(s) within the manifold hub 20. The transparent material may be a plastic, such as ABS plastics or other known plastic materials. As illustrated in Figures 1-8, an embodiment of the manifold hub 20 may have a substantially "F" shape.

As shown in Figures 2A, 2B and 4, the manifold hub 20 includes a first port 30 for connecting to an infusion tube 34 through which materials including wash fluids, diagnostic agents or treatment agents are delivered from an infusion device 38 to the first port 30, the manifold hub 20 and, eventually, the ductal access catheter 40. The connected infusion device 38 may include a syringe or other known fluid containers. The infusion device 38 may include a fluid receptacle, such as a bag or a container, positioned at a location above the breast of the patient. The height of the container above the breast of the patient and gravity are used to deliver the fluid from the infusion device 38 to the infusion tube 34. Figures 2A and 2B do not form part of the present invention.

As shown in Figures 2A, 2B and 4, the manifold hub 20 also includes a second port 32 for connection to a collection tube 36. Ductal fluid samples collected from within the breast duct may be delivered from the manifold hub 20 to a collection receptacle via the collection tube 36. The collection receptacle may include a syringe or other known fluid collection device including a medical fluid bag or container. The collection receptacle may include or be connected to a source of negative pressure so that an area of low pressure may be created within the collection tube 36 and, if needed, the manifold hub 20 for assisting in the delivery of the retrieved ductal fluid sample to the collection receptacle. The area of low pressure, for example a vacuum, may be created using a bulb syringe, a hand-operated vacuum source, a foot operated vacuum source or motor controlled vacuum source. These vacuum sources may include a pump that creates negative pressure within the collection tube 36. In addition to a source of lower pressure, or in place of the source of lower pressure, low pressure within collection tube 36 may be created by infusing fluid into the manifold hub 20, thereby increasing the pressure within the manifold hub 20 relative to the collection tube 36. In any of these constructions, the collection receptacle may be positioned at a location below the patient during the procedure so that the collected ductal fluid sample may be delivered to the receptacle by gravity. Figures 2A and 2B do not form part of the present invention.

The first and second ports 30, 32 may include an opening along the sidewall of the manifold hub 20 that is round, oval or any other geometric shape conducive to fluid flow either into the duct or out from the duct as shown in Figures 2A and 2B. The diameter of the ports 30, 32 may be that diameter which is suitable to achieve a desired flow rate into the duct or aspiration or collection rate out from the duct. Thus, the diameters of the ports 30, 32 may be in a range from about 0.060 inches to about 0.090 inches. One side port 30, 32 may be larger or smaller than the other, especially where such differential port size provides a desired flow rate into or out from one of the lumens, or an overall lavage efficiency of infusion and aspiration or collection of lavage and ductal fluid. Figures 2A and 2B do not form part of the present invention.

Figures 1-6 illustrate that the first and second ports 30, 32 and their associated tubes 34, 36, respectively, are positioned within a connector housing 25 that extends transverse to the longitudinal axis of the manifold hub 20 and the catheter 40. The connector housing 25 may be integrally formed with the manifold hub 20 as a unitary element. Alternatively, the connector housing 25 may be formed separate of the manifold hub 20 and secured to the manifold hub 20 as discussed below with respect to the catheter 40. The connector housing 25 includes two channels 26 that each receives one of the tubes 34, 36. The channels 26 extend from the ports 30, 32 on the manifold hub 20 to the outer, end surface of the connector housing 25. Each channel 26 aligns the received tube 34, 36 with its respective manifold hub port 30, 32 for easy, reliable and quick connection of the tubes 34, 36 to their respective ports 30, 32. The channels 26 also support the tubes 34, 36 at their point of connection to their ports 30, 32 so that the tubes 34, 36 do not create a moment (that may torque the duct) about the point where they connect to their respective ports 30, 32. The connector housing 25 may also include contoured sidewalls 28 with integrally formed, or otherwise secured, ridges 29 that may be gripped by an attendant or a practitioner. The contoured sidewalls 28 permit easy grasping by the attendant or practitioner and allow the attendant or practitioner to orient the instrument 10 during a procedure without looking at the instrument 10.

As shown in Figure 2B, the first and second ports 30, 32 may include posts 35 that extend within connector housing 25 and receive the flexible infusion and collection tubes 34, 36. The infusion and collection tubes 34, 36 may be formed of flexible tubing such as surgical tubing. However, the tubes 34, 36 may be formed of any flexible material including a flexible plastic. In one embodiment, the tubes 34,36 are formed of flexible PVC. Figures 2A and 2B do not form part of the present invention.

The posts 35 may securely receive the tubes 34, 36 when the tubes 34, 36 are positioned over, or within, the posts 35. Alternatively, the ports 30, 32 and their associated posts 35 may include luer lock fittings (not shown) that cooperate with corresponding luer lock fittings on a first end of the tubes 34, 36. The second end of the tubes 34, 36 may also include luer lock fittings that mate with standard luer lock fittings on the syringes or other fluid containers. The luer lock fittings may be either male or female fittings. As discussed herein, the syringes and other fluid containers may carry and infuse saline, diagnostic materials, such as contrast materials, and therapeutic treatment materials into the infusion tube 34. The tubes 34, 36 may be secured to the posts 35 of the manifold hub 20 and the luer locks using a UV curable adhesive or other known bonding agents. Alternatively, the tubes 34, 36 may be secured to the manifold hub 20 and the luer locks by overmolding.

As shown in Figures 1-6, the first port 30 may be positioned adjacent the second port 32 along the perimeter of the manifold hub 20. In the illustrated embodiment, the circumferentially adjacent ports 30, 32 are spaced the same longitudinal distance from the first and second ends 22, 24 of the manifold hub 20. This spacing of the ports 30, 32 provides for a compact and low profile manifold hub 20 that, as discussed above, will not create a moment and associated forces that toque the duct when the manifold hub 20 is positioned on the patient and free of support from a practitioner or other attendant.

The second port 32 may be circumferentially spaced any distance from the first port 30 around the wall of the manifold hub 20. The first port 30 may be between forty-five and ninety degrees offset from the second port 32 around the circumference of the manifold hub 20. Alternatively, the first port 30 may be circumferentially offset along the manifold wall from the second port 32 by between ninety and one hundred-eighty degrees. The first port 30 is circumferentially offset from the second port 32 by about one hundred-eighty degrees so that the first and second ports 30, 32 oppose each other within the manifold hub 20.

Alternatively, it is possible for the second port 32 to be located along the hub 20 at a position that is spaced a greater longitudinal distance away from the catheter 40 than the first port 30. The second port 32 may be used to collect the fluid that enters the manifold hub 20 from the collection catheter 40. For example one port may be located about 2.0 cm from the distal tip of the catheter 40 and one port may be located about 2.5 cm from the distal tip of the catheter 40.

The tubes 34 and 36 may each include a one-way check valve 39 (Figure 2A) to control the fluid flow into and out of the manifold hub 20. The check valve 39 in the tube 34 may prevent, for example, wash fluid from flowing back into a syringe connected to tube 34 after being infused into tube 34. Similarly, check valve 39 in tube 36 may be used to prevent retrieved ductal fluid samples in tube 36 from flowing back into the manifold hub 20. In an alternative embodiment, pinch clamps on the tubes 34, 36, may replace one or both of the check valves 39. For example, a check valve 39 may be positioned within the infusion tube 34 and a conventional pinch clamp may be positioned on the collection tube 36. Other known devices for controlling the direction and timing of fluid flow within a tube 34, 36 may also be used.

As shown in Figures 1-6, the catheter 40 includes a thin walled microcatheter 41 that is secured to the manifold hub 20. In a first construction, the microcatheter 41 is integrally formed as part of the manifold hub 20. In another construction, the microcatheter 41 is formed as a separate piece and then secured to the manifold hub 20 by microwelding or a UV curable adhesive. Other known techniques for securing the microcatheter 41 to the manifold hub 20 could be used. Moreover, the catheter 40 may be coated with a known agent to provide a lubricious coating that allows it to be easily introduced into the breast duct openings. The coating may include a lubricant, a cleaning agent, anesthetic and/or a dilating agent. The microcatheter 41 may be formed of any known biocompatible material such as FEP. The catheter 40 may have an outer diameter in a range from about 0.01inch (about 0.25 mm) to about 0.05 inch (about 1.25 mm) with an inner lumen 43 having a diameter in the range from about 0.008 inch (about 0.2 mm) to about 0.047 inch (about 1.2 mm). Furthermore, the microcatheter 41 may have an inner lumen 43 having an outer diameter of about 0.030 inch (about 0.762 mm) and an inner diameter of about 0.025 inch (about 0.63 mm).

The catheter 40 may include length indicia (not shown) on an outer surface of the catheter 40 that permits a user to determine the depth to which the distal end of the catheter has been introduced into the breast duct. Alternatively, the catheter 40 could include an integrally formed or attached stop element (not shown) that prevents insertion of the catheter into the duct beyond a predetermined distance. The stop element may comprise a knob on the catheter 40 having an increased diameter for preventing the distal portion of the catheter 40 from entering a duct a greater distance than the knob is spaced from the distal end of the catheter40.

As illustrated in Figures 1-7, the catheter 40 may be tapered along its length to make a smooth transition with a received introducer 50 so that a perceptible transition between the catheter 40 and the introducer 50 that would cause any pain to the patient is not formed and felt by the patient. The catheter 40 may also include an atraumatic distal tip portion 42 at its distal end. The distal tip portion 42 may be tapered, contoured and/or rounded so as to produce an atraumatic tip that will reduce or eliminate trauma to the duct upon entry through the ductal orifice and introduction into the ductal lumen past the ductal sphincter. The distal tip portion 42 may also reduce or eliminate trauma upon withdrawal of the catheter 40 from the duct after the medical procedure, such as ductal lavage or the infusion of a diagnostic and/or treatment agent, has been completed. The tip portion 42 may be composed of a soft polymeric material, e.g. including polyvinyl chloride, polyethers, polyamides, polyethylenes, polyurethanes, copolymers thereof and the like. The tip portion 42 may have a diameter in the range from about 0.012 inches (about 0.031 mm) to about 0.020 inches (about 0.051 mm). In a construction, the tip portion 42 has a diameter in the range from about 0.014 inches (about 0.036 mm) to about 0.018 inches (about 0.046 mm). The length of the tip portion 42 (extending from the distal end of the distal portion of the catheter 40 toward the proximal end of the catheter 40) may be in a range from about 0.10 inch (about 0.25 cm) to about 1.0 inch (about 2.5 cm), more typically in the range from about 0.20 inch (about 0.50 cm) to about 0.70 inch (about 1.8 cm).

The stiffened distal portion of the catheter 40, including the distal tip 42, may have an average bending stiffness in the range from about 0.001 cm-kg (0.010 inch-lbs) to about 0.576 cm-kg (0.5 inch-lbs). The catheter 40 may also have a stiffness that is similar to that of a heavy suture (approximately 0.025 OD). The proximal portion of the catheter 40 may have a cross-sectional geometry and/or size that inhibits insertion through the ductal orifice and into the ductal lumen.

A Touhy-Borst fitting 70 may be positioned at a proximal end 22 of the manifold hub 20 to allow a user to easily receive and move the catheter 40 over an introducer 50 as shown in Figures 1-6. The Touhy-Borst fitting 70 is positioned at the end of the manifold hub 20 to cover and seal the opening 77 through which an introducer 50 (discussed-below) including a guidewire, stylet, dilator or the like may extend. The Touhy-Borst fitting 70 comprises a silicone plug 72 including a small aperture 74 for receiving the introducer 50, and a threaded cap 76. When the cap 76 is rotated in a first direction, the silicone plug 72 is altered and the size of the aperture 74 is reduced. This results in the silicone plug 72 forming a seal around the inserted introducer 50. When the cap 76 is turned in a second, opposite direction, the aperture 74 and created seal open, thereby allowing the introducer 50 to be removed. The silicone plug 72 may also be closed to seal the proximal end of the manifold hub 20 so when the introducer 50 is not present so that the distal end of the manifold hub 20 is sealed against fluid flow when the proximal end 22 is free of an introducer 50.

The introducer 50 may be located within the manifold hub 20 to assist in placing the catheter 40 into the breast duct and ductal lumen via the ductal opening as shown in Figure 1. The introducer 50 may include a tapered dilator, a series of progressively larger diameter dilators, a guidewire, including tapered guidewires, a stylet or other known introducers. As illustrated, the introducer 50 will pass through the Touhy-Borst fitting 70 at the proximal end 22 of the manifold hub 20 so that the introducer 50 may be removed after positioning of the catheter 40 and prior to the infusion/collection of the wash fluid. As discussed above, prior to being inserted into the breast duct, the Touhy-Borst fitting 70 may be turned down over the introducer 50 during introduction and then backed off when the catheter 40 has been positioned within the breast duct to the desired depth. The introducer 50 may be formed of a stiff material such as a metal wire or a flexible plastic cord. Furthermore, the introducer 50 may be formed of stainless steel or a flexible material such as polypropylene monofilament. Alternatively, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. The introducer 50 may be coated with a liquid or dry lubricant material that reduces the friction between the introducer 50 and the breast duct during the introduction and advancement of the introducer 50 in the duct.

The introducer 50 may be made of metal or plastics, including shape memory metals and plastics, and may have a tapered and/or an atraumatic tip for gently probing and accessing a breast duct Preferably, a tapered tip 52 will extend distally of the catheter 40 during the introduction of the catheter 40 into the breast duct. After access of the duct is complete, the introducer 50 may be withdrawn, the Touhy-Borst fitting 70 may be closed and the indwelling catheter 40 may be positioned at a location distal to the ductal sphincter. The introducer 50 may have an outer diameter of from about 0.13 mm (0.005 inch) to about 0.76 mm (0.030 inch). In a construction, the introducer 50 has an outer diameter of about 0.25 mm (0.010 inch). The introducer 50 may extend through the manifold hub 20 and the lumen of the catheter 40. The introducer 50 may be tapered over its length.

During the process of introducing the catheter 40 into the duct, a ductal opening is located on the surface of a nipple by a practitioner or attendant and a first introducer 50 is advanced through the ductal opening into the duct The introducer 50 may be a long flexible guide wire, a shorter dilator or any of the other above-mentioned introducers. Prior to, or after the introducer 50 is positioned within the duct, the manifold hub 20 and catheter 40 may receive the first or a second introducer 50. As previously discussed, the Touhy-Borst fitting 70 may be locked about the received introducer 50 to form a fluid tight seal at the distal end of the manifold hub 20 so that fluid does not exit the manifold hub 20 around the introducer 50 during the insertion catheter 40 into the duct (See Figures 9, 10, 12 and 13).

When the catheter 40 is positioned within the duct as intended, the Touhy-Borst fitting 70 is opened and the introducer 50 removed (See Figure 14). The Touhy-Borst 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

Figures 31E-H are schematic diagrams illustrating an alternative construction of a ductal access device. In a preferred embodiment of the invention, the device includes a manifold hub 20 and a ductal access catheter 40 that extends from a distal end 21 of the manifold hub 20 with a centrally disposed single lumen which extends the length of the catheter 40. Catheter 40 includes a tubular introducer pathway adapted to slideably receive a ductal introducer 50 therein. For ease of explanation, as used herein the term "introducer" is defined to include guidewires, dilators, stylettes or portion of any of these that may be inserted within a ductal orifice or a passageway of a catheter sized to access a breast duct.

As illustrated, the manifold hub 20 may have a low profile (height) in a direction that extends parallel to the length of the catheter 40. In another construction, hub 20 has a low profile so as to reduce the torque on the breast nipple after insertion of catheter 40. This overcomes the excessive generated torque on the breast nipple known to cause obstruction of ductal fluid due to compression of the tissue. Thus, improved collection of ductal cellular material is provided.

The manifold hub 20 is coupled to the proximal end 45 of the ductal access catheter 40. In a preferred construction, hub 20 includes transparent material so that the user may visualize the flow to and from the breast duct during a lavage procedure. The transparent material may be plastic, such as ABS plastics or other known plastic materials. As illustrated in Figures 31E-H, a construction of the manifold hub 20 may have a substantially "U" shape.

A fitting 70 may be positioned at a proximal end 22 of the manifold hub 20 to allow a user to easily receive and move the catheter 40 over an introducer 50. The fitting 70 is positioned at the end of the manifold hub 20 to cover and seal the opening 77 through which an introducer 50 including a guidewire, stylet, dilator or the like may extend. The fitting 70 comprises a silicone gasket including a small aperture 74 for receiving the introducer 50, and a cap 76. The cap 76 locks the silicon gasket to the manifold hub 20 thus maintaining a seal between the hub and the gasket. The gasket may be made of any suitable flexible biocompatible material. Around the neck of the silicone gasket is a pair of interlocking hooks 110 which at the terminus of flexible side arms 100 which are part of the main body of the manifold hub 20. Located between the flexible side arms and the manifold hub are one or more springs 120 that force the flexible arms 100 away from the main body of the manifold hub 20. As the springs 120 force the flexible side arms 100 away from the manifold hub 20, the interlocking hooks 110 at the end of the flexible side arms 100 apply pressure to opposite sides of the fitting 70. This results in the fitting 70, which may be comprised of silicone or any other flexible material, to compress around the aperture 74 forming a seal around the inserted introducer 50. When pressure is applied to the flexible arms 100 thus compressing the springs 120, the interlocking hooks 110 are actuated apart thus releasing pressure on the silicon gasket and thus breaking seal around the inserted introducer 50. The flexible side arms 100 and the interlocking hooks 110 may be constructed of metal or plastic or a combination thereof.

In an alternative construction, the manifold hub 20 is comprised of a single flexible arm 100 with a single hooked end 110 which, when activated, opens or closes the fitting 70.

The introducer 50 may be located within the manifold hub 20 to assist in placing the catheter 40 into the breast duct and ductal lumen via the ductal opening as shown in Figures 9, 10, 12 and 13. The introducer 50 may include a tapered dilator, a series of progressively larger diameter dilators, a guidewire, including tapered guidewires, a stylet or other known introducers. As illustrated, the introducer 50 will pass through the fitting 70 at the proximal end 22 of the manifold hub 20. The introducer of the present invention may consist of two parts, a wire and a ring shaped handle located at the end of the introducer. The introducer 50 has a diameter equal to or slightly less than the ID of the catheter 40. The length of the introducer 50 may extend approximately 20 -30 mm beyond the distal end of the catheter 40 when the introducer is fully seated. The extended length of the introducer 50 beyond the distal end of the catheter 40 allows the introducer to be used as a dilator prior to the insertion of the catheter in the breast duct. As illustrated in Figure 18, the distal end of the introducer 50 may be tapered. The distal tip of the introducer 50 may be tapered to an OD in the range of 0.20 to 0.25 mm (0.008 to 0.010 inches) from the proximal end of the introducer that may have an OD equal to or slightly less than the ID of the distal end of the catheter 42. The small diameter allows for easier insertion of the introducer into the breast milk duct. The introducer 50 may be formed of a stiff material such as a metal wire or a flexible material such as plastic. Furthermore, the introducer 50 may be formed of Nitinol. Nitinol is preferred due to its flexibility, durability, and biocompatibility. The introducer 50 may be constructed from a Nitinol wire that is ground to include a tapered small diameter tip. Moreover, the introducer 50 may be formed of multiple materials or the same materials having different stiffnesses. As a result, the introducer 50 may have sections that are more flexible than adjacent sections of the same introducer 50. As a result, for example, the introducer 50 may have a first, stiff portion for guiding the introducer 50 within the ductal lumen and a second, flexible portion that allows the introducer 50 to conform to the shape of the ductal lumen or lumen branch into which it is introduced. In any of the above-discussed embodiments, the introducer 50 may be coated with a liquid or dry lubricant material that reduces the friction between the introducer 50 and the breast duct during the introduction and advancement of the introducer 50 in the duct

The introducer 50 may be made of metal or plastics, including shape memory metals and plastics, and may have a tapered and/or an atraumatic tip for gently probing and accessing a breast duct. Preferably, a tapered tip 52 will extend distally of the catheter 40 during the introduction of the catheter 40 into the breast duct. After access of the duct is complete, the catheter 40 may be slipped over the introducer 50 and positioned at a location distal to the ductal sphincter.

The proximal end of the introducer 50 may be formed into a ring shaped handle (see Figures 31E and 31G). The advantage of the ring shaped handle is that is allows the device 10 to be used with one hand. The ring shaped handle may allow the practitioner to grasp the flexible side arms 100 of the device 10 using their thumb and forefinger and to manipulate the introducer 50 by using their index finger which may be positioned through the ring shaped handle. The ring shaped handle may be constructed of a thermoplastic such as ABS or polycarbonate or pellathane, and may be shaped in alternative forms that will allow the practitioner to operate the device 10 with one hand.

During the process of introducing the catheter 40 into the duct, a ductal opening is located on the surface of a nipple by a practitioner or attendant and an introducer 50 is advanced through the ductal opening into the duct. The introducer 50 may be a long flexible guide wire, a shorter dilator or any of the other above-mentioned introducers. Prior to, or after the introducer 50 is positioned within the duct, the manifold hub 20 and catheter 40 may receive the introducer 50. As previously discussed, compressing the flexible side arms 100 thus actuating the interlocking side arms 110 away from the fitting 70 may open the fitting 70. Once pressure is released from the flexible side arms 100, the interlocking arms 110 are forced away from the manifold hub 20 by the springs 120 thus closing the aperture 74 about the received introducer 50 to form a fluid tight seal at the distal end of the manifold hub 20 so that fluid does not exit the manifold hub 20 around the introducer 50 during the insertion catheter 40 into the duct (See Figures 9, 10, 12, 13, 31E, and 31G).

When the catheter 40 is positioned within the duct as intended, the fitting 70 is opened and the introducer 50 removed. The 70 may then be closed again to seal the hub 20. Fluid is then introduced into the manifold hub 20, through the catheter 40 and into the breast duct until resistance is met during the infusion. At this time, it is assumed that the duct is filled. The infusion tube, for example tube 34, may then be closed and the fluid allowed to remain in the duct for a preselected time. During this preselected time, the breast may be massaged and squeezed to stimulate mixing of the wash fluid and ductal fluid, and also ultimately to encourage the fluid to leave the duct and enter the manifold hub 20. The collection tube, for example tube 36, may be opened and the breast squeezed to urge the fluid to progress through the catheter 40 and into the manifold hub 20. If desired, when cloudy return fluid is seen in the hub 20 (which may be transparent or include a transparent window), the infusion tube 34 may be opened and fluid infused into the manifold hub 20 to push the ductal fluid sample that has collected in the hub 20 into the collection tube 36 and a waiting collection receptacle. Alternatively, and possibly additionally, aspiration pressure may be applied within the manifold hub 20 and at the collection tube 36 to aspirate any fluid remaining in the hub 20 into the collection receptacle. The process is repeated either following another infusion of fluid into the duct or by another round of squeezing to encourage return and collection of the infused fluid and cellular material from within the breast duct.

A method of lavage may include seating a patient substantially upright in a chair during the lavage procedure, rather than the standard or classic supine (face up) position. Alternatively, the patient may be lavaged in a prone position, face down, with nipples and breast down. The prone face down position takes advantage of gravity and allows the breast ducts to drain into the collection receptacle during the procedure when the outflow port is open. Thus, as discussed above, the lavaging procedure may include infusing the breast duct with a wash fluid through an open inflow lumen while an outflow lumen is closed; closing the inflow lumen when the duct is filled; squeezing or massaging the breast or both; and opening the outflow lumen to collect the wash fluid.

The cells collected may comprise ductal epithelial cells; the ductal fluid collected may comprise molecular and cellular material. Analysis of the ductal epithelial cells and/or the molecular and cellular material in the ductal fluid may proceed as described below discussing the diagnostic methods possible of these collected materials. The collected cells and fluid and fluid components may be analyzed. The lavage fluid including the ductal cells may be analyzed for diagnostic purposes. Conditions in a breast milk duct that are desirable to diagnose include a cancer or precancer condition. The precancer condition may include atypical ductal hyperplasia (ADH) or low-grade ductal carcinoma in situ (LG-DCIS). The diagnostic agent may also have the ability to diagnose other breast related conditions, including, e.g. fibrotic, cystic or conditions relating to lactation. Diagnostic agents may be mixed with the ductal fluid (either in the lavage procedure, or after the fluid is collected).

The fluid infused into the duct to lavage the duct may include known, biocompatible fluids. These lavage fluids may include saline, phosphate buffered saline, a nonabsorbable fluid, an isotonic solution, an osmotic solution, a hypotonic solution, and a hypertonic solution. The wash fluid may comprise for example, saline, phosphate buffered saline, a nonabsorbable fluid, an isotonic solution, an osmotic solution, a hypotonic solution, a hypertonic solution.a protein, a colloid, a sugar, a polymer, mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, a synthetic colloid, an antibody, a binding protein, or albumin.

As mentioned above, a diagnostic or therapeutic agent may be introduced into a breast duct through the manifold hub 20 and catheter 40. The introduced agent for infusing into the duct may comprise a non-absorbable fluid and/or an oncotic agent and/or an osmotic agent. The agent may be soluble. The agent may comprise a molecule that is a protein, a colloid, a sugar, or a polymer. The agent may be mannitol, sorbitol, glucose, glycerol, sucrose, raffinose, fructose, lactulose, sodium chloride, polyethyleneglycol (PEG), maltodextrin, dextran (e.g. dextran 70), hydroxyethyl starch, fluid gelatin, or a synthetic colloid. The agent may comprise a protein and the protein may be a binding protein or an antibody. The binding protein may be albumin. Administering may comprise administering locally, and local administration may comprise administering intraductally. A system for increasing or standardizing an amount of fluid collectable from a milk duct of a breast may comprise infusing a nonabsorbable fluid and/or an osmotic agent and/or an oncotic agent into the ductal lumen, a medical tool for delivering the agent to the ductal lumen, and instructions for use.

## Claims

1. A device (10) for being introduced and positioned within a breast duct for introducing or removing material within the breast duct, said apparatus comprising:
a manifold hub (20) comprising a plurality of port openings (30, 32) in fluid communication with an interior of said manifold hub, at least two of said openings being in fluid communication with a pair of elongated channels (26) that extend through a portion of said manifold hub;
a catheter (40) extending from the manifold hub, said catheter being sized and configured for positioning within a breast duct; and
a retractable spacer (90) that may be moved in a direction parallel to the length of the catheter to space the manifold hub a distance above the surface of a nipple,
wherein said catheter includes a longitudinal axis; and said manifold hub includes a height that extends substantially parallel to said longitudinal axis of said catheter and a length that extends substantially perpendicular to said longitudinal axis of said catheter, wherein said length of said manifold hub is greater than the height of said manifold hub.

2. The device (10) of claim 1 wherein said plurality of ports (30, 32) of said manifold hub (20) comprises at least four ports in fluid communication with an interior of said manifold hub, and wherein said pair of channels (26) form a portion of said manifold hub.

3. The device (10) of claim 1 wherein said catheter (40) extends from a first end of said manifold hub, and further comprising a sealing fitting (70) positioned at a second end (22) of said manifold hub opposite said catheter, said sealing fitting including an aperture (74) that may be repeatedly opened and closed.

4. The device (10) of claim 3 wherein said sealing fitting (70) includes a Touhy-Borst fitting for sealing about a member extending through said manifold hub (20) at said second end (22) of said manifold hub.

5. The device (10) of claim 4 wherein said Touhy-Borst fitting (70) comprises a silicone plug (72) comprising said aperture (74) and a threaded cap (76) such that when said cap is turned in a first direction, the silicone plug is altered and a size of said opening within said silicone plug is reduced, thereby forming a seal about said member extending through said manifold hub (20).

6. The device (10) of claim 1 further including an anchoring member (11) secured to manifold hub (20) for securing the apparatus to the body of a patient.

7. The device (10) of claim 6 wherein said anchoring member (11) has a lower surface for contacting the body of the patient, and said lower surface includes a biocompatible adhesive.

8. The device (10) of claim 1 further comprising a source of negative pressure in communication with said manifold hub (20) for directing a ductal fluid sample from within said manifold hub to a ductal fluid collection receptacle (39).

9. The device (10) of claim 8 wherein said source of negative pressure comprises a powered vacuum source in fluid communication with said manifold hub (20).

10. The device (10) of claim 8 wherein said source of negative pressure comprises a mechanically controlled negative pressure source in fluid communication with said manifold hub (20):

11. The device (10) of claim 1 further comprising a fluid container for holding a fluid being delivered to the breast duct, a fluid infusion member (38) for delivering fluid from said fluid container to said manifold hub (20), a ductal fluid collection receptacle and a ductal fluid collection member extending between said manifold hub (20) and said collection receptacle for delivering ductal fluid samples from said manifold hub to said collection receptacle.

12. The device (10) of claim 11 wherein said fluid container includes a fluid bag for housing a biocompatible fluid, said fluid bag being positionable vertically above said manifold hub (20) for delivering the biocompatible fluid to said manifold hub.

13. The device (10) of claim 11 wherein said fluid infusion member (38) includes a syringe.

14. The device (10) of claim 11 wherein said ductal fluid collection receptacle includes a syringe.

15. The device (10) of claim 11 wherein said ductal fluid collection receptacle (39) includes a fluid collection bag for being vertically spaced below said manifold hub (20) to receive a ductal fluid sample from within said manifold hub.

16. The device (10) of claim 11 wherein said fluid infusion member (38) and/or said ductal fluid collection member includes a one-way check valve (39).

17. The device (10) of claim 1 wherein said catheter (40) includes a microcatheter (41) having a lubricious coating that allows it to be easily introduced into the breast duct openings.

18. The device (10) of claim 17 wherein said lubricious coating may include a lubricant, a cleaning agent, anesthetic and/or a dilating agent.

19. The device (10) of claim 17 wherein a proximal end of said microcatheter (41) includes an atraumatic tip.

20. The device (10) of claim 1 wherein said manifold hub (20) is formed of a transparent material.

21. The device (10) of claim 20 wherein said transparent material includes an ABS plastic.

22. The device (10) of claim 1 wherein said spacer (90) comprises at least two telescoping members (92, 93) extending between the manifold hub (20) and a portion of the catheter (40).

23. The device (10) of claim 1 wherein said spacer (90) includes a member (95) that is moveable relative to the manifold hub (20) such that a portion of the manifold hub is received within said moveable member.

24. The device (10) of claim 1 wherein said manifold hub (20) includes vertical sidewalls (28) with recesses (29) for receiving fingers of a practitioner or attendant for the easy manipulation of said manifold hub relative to the breast.

## Patentansprüche

1. Vorrichtung (10) zum Einführen und Anordnen innerhalb eines Brustgangs zum Einführen oder Sammeln von Material innerhalb des Brustgangs, die Vorrichtung umfasst:
eine zentrale Verteilerstelle (20) umfassend eine Vielzahl von Anschlussöffnungen (30, 32) in Flüssigkeitsaustausch mit einem Inneren der zentralen Verteilerstelle, mindestens zwei der Öffnungen befinden sich in Flüssigkeitsaustausch mit einem Paar von verlängerten Kanälen (26), die sich durch einen Abschnitt der zentralen Verteilerstelle erstrecken;
einen Katheter (40), der sich von der zentralen Verteilerstelle erstreckt, der Katheter ist bezüglich seiner Größe und Gestaltung derart, dass er innerhalb eines Brustgangs angeordnet werden kann; und
einen einschiebbaren bzw. ausfahrbaren Abstandshalter, der in einer Richtung parallel zu der Länge des Katheters bewegt werden kann, um die zentrale Verteilerstelle ein Stück oberhalb der Oberfläche einer Brustwarze zu beabstanden,
wobei der Katheter eine Längsachse einschließt; und die zentrale Verteilerstelle eine Höhe, die sich im Wesentlichen parallel zu der Längsachse des Katheters erstreckt und eine Länge, die sich im Wesentlichen senkrecht zu der Längsachse des Katheters erstreckt, einschließt, wobei die Länge der zentralen Verteilerstelle länger ist als die Höhe der zentralen Verteilerstelle.

2. Vorrichtung (10) nach Anspruch 1, wobei die Vielzahl der Anschlüsse (30, 32) der zentralen Verteilerstelle (20) mindestens vier Anschlüsse in Flüssigkeitsaustausch mit einem Inneren der zentralen Verteilerstelle aufweist, und wobei das Paar der Kanäle (26) einen Abschnitt der zentralen Verteilerstelle bilden.

3. Vorrichtung (10) nach Anspruch 1, wobei der Katheter (40) sich von einem ersten Ende der zentralen Verteilerstelle erstreckt, und weiterhin eine Dichtungsverbindung (70) aufweist, die an einem zweiten Ende (22) der zentralen Verteilerstelle gegenüber des Katheters angeordnet ist, die Dichtungsverbindung schließt eine Öffnung (74) ein, die wiederholt geöffnet und geschlossen werden kann.

4. Vorrichtung (10) nach Anspruch 3, wobei die Dichtungsverbindung (70) eine Touhy-Borst-Verbindung einschließt, um ein Element abzudichten, das sich durch die zentrale Verteilerstelle (20) an dem zweiten Ende (22) der zentralen Verteilerstelle erstreckt.

5. Vorrichtung (10) nach Anspruch 4, wobei die Touhy-Borst-Verbindung (70) einen Silikonanschluss (72) umfasst, der die Öffnung (74) und einen Einschraubstutzen bzw. eine einschraubbare Verschlusskappe (76) umfasst, so dass, wenn die Verschlusskappe in eine erste Richtung gedreht wird, der Silikonanschluss verändert wird und eine Größe der Öffnung innerhalb des Silikonanschlusses verringert wird, wodurch sich eine Dichtung um das erste Element bildet, die sich durch die zentrale Verteilerstelle (20) erstreckt.

6. Vorrichtung (10) nach Anspruch 1, die weiterhin ein Verankerungselement (11) einschließt, das an der zentralen Verteilerstelle (20) gesichert ist, um den Apparat an dem Körper eines Patienten zu sichern.

7. Vorrichtung (10) nach Anspruch 6, wobei das Verankerungselement (11) eine untere Oberfläche zum Inkontaktbringen mit dem Körper des Patienten aufweist, und die untere Oberfläche schließt einen bioverträglichen Klebstoff ein.

8. Vorrichtung (10) nach Anspruch 1, weiter umfassend eine negative Druckquelle in Verbindung mit der zentralen Verteilerstelle (20) um eine Gangflüssigkeitsprobe von innerhalb der zentralen Verteilerstelle zu einem Sammelbehälter (39) für die Gangflüssigkeit zu leiten.

9. Vorrichtung (10) nach Anspruch 8, wobei die negative Druckquelle eine angetriebene Vakuumquelle in Flüssigkeitsaustausch mit der zentralen Verteilerstelle (20) umfasst.

10. Vorrichtung (10) nach Anspruch 8, wobei die negative Druckquelle eine mechanisch kontrollierte negative Druckquelle in Flüssigkeitsaustausch mit der zentralen Verteilerstelle (20) umfasst.

11. Vorrichtung (10) nach Anspruch 1, weiter umfassend einen Flüssigkeitsbehälter zum (Zurück)Halten einer Flüssigkeit, die in den Brustgang abgegeben werden soll, ein Flüssigkeitsinfusionselement (38) zum Abgeben von Flüssigkeit aus dem Flüssigkeitsbehälter an die zentrale Verteilerstelle (20), einen Sammelbehälter für Gangflüssigkeit und ein Sammelelement für Gangflüssigkeit, das sich zwischen der zentralen Verteilerstelle (20) und dem Sammelbehälter zum Abgeben von Gangflüssigkeitsproben von der zentralen Verteilerstelle an den Sammelbehälter erstreckt.

12. Vorrichtung (10) nach Anspruch 11, wobei der Flüssigkeitsbehälter einen Flüssigkeitsbeutel zum Aufnehmen einer bioverträglichen Flüssigkeit einschließt, der Flüssigkeitsbeutel ist vertikal oberhalb der zentralen Verteilerstelle (20) anordenbar, um die bioverträgliche Flüssigkeit an die zentrale Verteilerstelle abzugeben.

13. Vorrichtung (10) nach Anspruch 11, wobei das Flüssigkeitsinfusionselement (38) eine Spritze einschließt.

14. Vorrichtung (10) nach Anspruch 11, wobei der Sammelbehälter für Gangflüssigkeit eine Spritze einschließt.

15. Vorrichtung (10) nach Anspruch 11, wobei der Sammelbehälter für Gangflüssigkeit (39) einen Flüssigkeitssammelbeutel einschließt, der vertikal unterhalb der zentralen Verteilerstelle (20) beabstandet angeordnet werden kann, um eine Gangflüssigkeitsprobe von innerhalb der zentralen Verteilerstelle aufzunehmen.

16. Vorrichtung (10) nach Anspruch 11, wobei das Flüssigkeitsinfusionselement (38) und/oder das Gangflüssigkeitssammelelement ein Einwegabsperrventil (39) einschließt.

17. Vorrichtung (10) nach Anspruch 1, wobei der Katheter (40) einen Mikrokatheter (41) mit einer gleitfähigen Beschichtung einschließt, die es erlaubt, dass er einfach in die Brustgangöffnungen eingeführt werden kann.

18. Vorrichtung (10) nach Anspruch 17, wobei die gleitfähige Beschichtung ein Gleitmittel, ein Reinigungsmittel, ein anästhetisches und/oder ein dehnendes bzw. weitendes Mittel einschließt.

19. Vorrichtung (10) nach Anspruch 17, wobei ein proximales Ende des Mikrokatheters (41) eine atraumatische Spitze einschließt.

20. Vorrichtung (10) nach Anspruch 1, wobei die zentrale Verteilerstelle (20) aus einem transparenten Material gebildet ist.

21. Vorrichtung (10) nach Anspruch 20, wobei das transparente Material eine ABS Plastik einschließt.

22. Vorrichtung (10) nach Anspruch 1, wobei der Abstandshalter (90) mindestens zwei Teleskopelemente (92, 93) umfasst, die sich zwischen der zentralen Verteilerstelle (20) und einem Abschnitt des Katheters (40) erstrecken.

23. Vorrichtung (10) nach Anspruch 1, wobei der Abstandshalter (90) ein Element (95) einschließt, das relativ zu der zentralen Verteilerstelle (20) beweglich ist, so dass ein Abschnitt der zentralen Verteilerstelle innerhalb des beweglichen Elements aufgenommen wird.

24. Vorrichtung (10) nach Anspruch 1, wobei die zentrale Verteilerstelle (20) vertikale Seitenwände (28) mit Ausnehmungen (29) zum Aufnehmen von Fingern eines Arztes oder eines Pflegers für die einfache Bedienung der zentralen Verteilerstelle in Bezug auf die Brust einschließt.

## Revendications

1. Dispositif (10) à introduire et placer à l'intérieur d'un canal mammaire pour introduire ou retirer un matériel à l'intérieur du canal mammaire, ledit appareil comprenant :
un raccord collecteur (20) comprenant une pluralité d'ouvertures (30, 32) en communication fluidique avec un intérieur dudit raccord collecteur, au moins deux desdites ouvertures étant en communication fluidique avec une paire de canaux allongés (26) qui s'étendent à travers une partie dudit raccord collecteur ;
un cathéter (40) s'étendant depuis le raccord collecteur, ledit cathéter étant dimensionné et configuré pour se placer à l'intérieur d'un canal mammaire ; et
un espaceur rétractable (90) qui peut être déplacé dans une direction parallèle à la longueur du cathéter pour écarter le raccord collecteur d'une distance au-dessus de la surface d'un mamelon,
dans lequel ledit cathéter comprend un axe longitudinal ; et ledit raccord collecteur a une hauteur qui s'étend de manière sensiblement parallèle audit axe longitudinal dudit cathéter et une longueur qui s'étend de manière sensiblement perpendiculaire audit axe longitudinal dudit cathéter, dans lequel ladite longueur dudit raccord collecteur est supérieure à la hauteur dudit raccord collecteur.

2. Dispositif (10) selon la revendication 1, dans lequel ladite pluralité d'ouvertures (30, 32) dudit raccord collecteur (20) comprend au moins quatre orifices en communication fluidique avec un intérieur dudit raccord collecteur, et dans lequel ladite paire de canaux (26) forme une partie dudit raccord collecteur.

3. Dispositif (10) selon la revendication 1, dans lequel ledit cathéter (40) s'étend depuis une première extrémité dudit raccord collecteur, et comprenant en outre un raccord d'étanchéité (70) placé au niveau d'une deuxième extrémité (22) dudit raccord collecteur, à l'opposé dudit cathéter, ledit raccord d'étanchéité comprenant une ouverture (74) qui peut être ouverte et fermée de manière répétitive.

4. Dispositif (10) selon la revendication 3, dans lequel ledit raccord d'étanchéité (70) comprend un raccord Touhy-Borst pour sceller autour un élément s'étendant à travers ledit raccord collecteur (20) au niveau de ladite deuxième extrémité (22) dudit raccord collecteur.

5. Dispositif (10) selon la revendication 4, dans lequel ledit raccord Touhy-Borst (70) comprend une fiche en silicone (72) comprenant ladite ouverture (74) et un bouchon fileté (76) de sorte que lorsque ledit bouchon est tourné dans une première direction, la fiche en silicone est modifiée et une taille de ladite ouverture à l'intérieur de ladite fiche en silicone est réduite, formant de ce fait un joint autour dudit élément s'étendant à travers ledit raccord collecteur (20).

6. Dispositif (10) selon la revendication 1, incluant en outre un élément d'ancrage (11) fixé au raccord collecteur (20) pour fixer l'appareil au corps d'un patient.

7. Dispositif (10) selon la revendication 6, dans lequel ledit élément d'ancrage (11) possède une surface inférieure destinée à entrer en contact avec le corps du patient, et ladite surface inférieure comprend un adhésif biocompatible.

8. Dispositif (10) selon la revendication 1, comprenant en outre une source de dépression en communication avec ledit raccord collecteur (20) pour diriger un échantillon de fluide canalaire ou depuis l'intérieur dudit raccord collecteur vers un réceptacle de recueil de fluide canalaire (39).

9. Dispositif (10) selon la revendication 8, dans lequel ladite source de dépression comprend une source de vide mécanisée en communication fluidique avec ledit raccord collecteur (20).

10. Dispositif (10) selon la revendication 8, dans lequel ladite source de dépression comprend une source de dépression contrôlée de manière mécanique en communication fluidique avec ledit raccord collecteur (20).

11. Dispositif (10) selon la revendication 1, comprenant en outre un récipient pour fluide destiné à contenir un fluide qui est délivré au canal mammaire, un élément de perfusion de fluide (38) pour délivrer un fluide depuis ledit récipient pour fluide jusqu'au raccord collecteur (20), un réceptacle de recueil de fluide canalaire et un élément de recueil de fluide canalaire s'étendant entre ledit raccord collecteur (20) et ledit réceptacle de recueil pour délivrer des échantillons de fluide canalaire depuis ledit raccord collecteur jusqu'au réceptacle de recueil.

12. Dispositif (10) selon la revendication 11, dans lequel ledit récipient pour fluide comprend une poche de fluide destinée à renfermer un fluide biocompatible, ladite poche de fluide étant positionnable verticalement au-dessus dudit raccord collecteur (20) pour délivrer le fluide biocompatible audit raccord collecteur.

13. Dispositif (10) selon la revendication 11, dans lequel ledit élément de perfusion de fluide (38) comprend une seringue.

14. Dispositif (10) selon la revendication 11, dans lequel ledit réceptacle de recueil de fluide canalaire comprend une seringue.

15. Dispositif (10) selon la revendication 11, dans lequel ledit réceptacle de recueil de fluide canalaire (39) comprend une poche de recueil de fluide pour être écarté verticalement en dessous dudit raccord collecteur (20) afin de recevoir un échantillon de fluide canalaire depuis l'intérieur dudit raccord collecteur.

16. Dispositif (10) selon la revendication 11, dans lequel ledit élément de perfusion de fluide (38) et/ou ledit élément de recueil de fluide canalaire comprend un clapet anti-retour unidirectionnel (39).

17. Dispositif (10) selon la revendication 1, dans lequel ledit cathéter (40) comprend un microcathéter (41) ayant un revêtement lubrifiant qui lui permet d'être aisément introduit dans les ouvertures d'un canal mammaire.

18. Dispositif (10) selon la revendication 17, dans lequel ledit revêtement lubrifiant peut comprendre un lubrifiant, un agent nettoyant, un agent anesthésique et/ou dilatant.

19. Dispositif (10) selon la revendication 17, dans lequel une extrémité proximale dudit microcathéter (41) comprend une pointe atraumatique.

20. Dispositif (10) selon la revendication 1, dans lequel ledit raccord collecteur (20) est formé d'une matière transparente.

21. Dispositif (10) selon la revendication 20, dans lequel ladite matière transparente comprend un plastique ABS.

22. Dispositif (10) selon la revendication 1, dans lequel ledit espaceur (90) comprend au moins deux éléments télescopiques (92, 93) s'étendant entre le raccord collecteur (20) et une partie du cathéter (40).

23. Dispositif (10) selon la revendication 1, dans lequel ledit espaceur (90) comprend un élément (95) qui est mobile par rapport au raccord collecteur (20) de sorte qu'une partie du raccord collecteur est reçue à l'intérieur dudit élément mobile.

24. Dispositif (10) selon la revendication 1, dans lequel ledit raccord collecteur (20) comprend des parois latérales verticales (28) avec des renfoncements (29) pour recevoir les doigts d'un praticien ou d'un assistant pour la manipulation facile dudit raccord collecteur par rapport au sein.
